# EUROPEAN PATENT APPLICATION

(11) **EP 3 831 378 A1**
(43) Date of publication of application: **09.06.2021**
(21) Application number: 20215763.2
(22) Date of filing: 08.04.2015
(51) Int. Cl.: A61K 31/397, A61K 9/20, A61P 21/00, A61P 25/28, A61P 37/00

(54) **SIPONIMOD IMMEDIATE RELEASE DOSAGE REGIMEN FOR TREATING AUTOIMMUNE DISEASES**

(30) Priority: 10.04.2014 US 201461977816 P
(62) Divisional of application: 15717256.0
(71) Applicant: Novartis AG, 4056 Basel (CH)
(72) Inventor: Bouillot, Philippe Michel René, 4002 Basel (CH); Dahlke, Frank, 4002 Basel (CH); Legangneux, Eric, 4002 Basel (CH); Reynaud, Emeric, 4002 Basel (CH); Wallstroem, Erik, 4002 Basel (CH)
(74) Representative: Bucher, Tamaris Clare

(57) **Abstract**

The present invention relates to siponimod (BAF312) for use in the treatment of an autoimmune disease, wherein an immediate release dosage form is administered once daily to a patient as maintenance regimen and wherein the patient has experienced a specific titration regimen with siponimod beforehand.

## Description

### Field of the invention

The present invention relates to siponimod (BAF312) for use in the treatment of an autoimmune disease, wherein an immediate release dosage form is administered and wherein patients are treated who have experienced a specific titration regimen with siponimod.

### Background

One of the most common inflammatory, demyelinating diseases of the central nervous system (CNS) is multiple sclerosis (MS), in which the insulating covers of nerve cells in the brain and spinal cord are damaged. MS takes several forms, with new symptoms either occurring in isolated attacks (relapsing forms) or building up over time (progressive forms). Between attacks, symptoms may go away completely. However, permanent neurological problems often occur, especially as the disease advances.

At the time of diagnosis, between 80% and 90% of MS patients have relapsing-remitting MS (RRMS). This form of MS is characterized by recurring relapses, i.e. acute episodes, of neurological symptoms. In around 80% of the patients with RRMS, this form then develops to secondary progressive MS (SPMS), around 19 years after disease onset. Progression proceeds with or without occasional relapses with minor remissions between relapses and is characterized symptomatically by continuous worsening of disability, independent of relapses. While anti-inflammatory therapies and immune modulation exert a beneficial effect in RRMS patients, they have little or no beneficial effect in the progressive stage of the disease. In patients with long disease duration the number of inflammatory infiltrates decreases, while neurodegeneration becomes a more prominent feature. Recently it has been discovered that inflammation in the brain does not only occur in RRMS patients, but also in SPMS patients. Furthermore, in patients with SPMS inflammation in the meninges can be found. Here it has been found that the extent of inflammation in the meninges correlates with the amount of neurodegeneration. Thus, inflammation seems to drive tissue degeneration at least in some patients.

Profound microglia activation at the sites of injury in the MS brain and the close association of these cells with degenerating oligodendrocytes and axons suggest that they play a key role in mediating demyelination and neurodegeneration. Many studies in vitro as well as in vivo suggest that microglial neurotoxicity might not only result from the presence of noxious stimuli but that it may also be caused by the absence of key molecules regulating microglial responses, e.g. neurotransmitters. In a normal condition intact neurons produce a large number of molecules with anti-inflammatory properties that could maintain the microglia cells in a hyporesponsive state. Hence, the loss of these molecules could remove this braking system and might permit microglia activation. It can be suggested that during the course of MS this source of restraint is gradually lost due to neuronal degeneration. Probably, this loss is still below a critical threshold in patients with RRMS and above this threshold when the disease changes to a secondary progressive course. Then, even minimal changes in the environment might be sufficient to lift the brakes on microglia cell activation. Under these conditions microglia cells would overreact to stimulation, upregulate the expression of neurotoxic molecules and eventually aggravate neuronal loss as long as the stimulation triggers are present.

Further, inflammation of the central nervous system (CNS) in SPMS and PPMS (primary progressive MS) differs from inflammation of RRMS. In RRMS at relapse, the blood-brain barrier is open, and large numbers of bloodborne T cells and monocytes/marcrophages enter the CNS parenchyma and locally release proinflammatory factors. In RRMS during remission, the blood-brain barrier is repaired, and the numbers of intraparenchymal T cells is dramatically decreased as is the degree of microglial cell activation. In progressive MS, inflammation is trapped behind a closed blood-brain barrier, and damage of the CNS parenchyma is provoked by the action of diffusible factors acting on microglia cells and a few intraparenchymal T cells. This finding is supported by the observation that in progressive MS lymph follicle-like structures are formed within the connective tissue compartments of the CNS, meninges and large perivascular spaces. It has been thus suggested that the failure of current immunosuppressive or immunomodulatory treatments in patients with SPMS and PPMS may be related to their inability to pass the blood-brain barrier and to reach therapeutically relevant concentrations within the CNS.

The relapses and remissions of RRMS that used to come and go change into a steady, gradual worsening of the disease leading to SPMS. Although RRMS can be unpredictable, the pattern of clear attacks followed by recovery typically is consistent. With SPMS, relapses tend to be less distinct. They may happen less often or not at all. When relapses do occur, their recovery normally is not as complete as in RRMS. Symptoms that indicate a shift towards SPMS include a steady increase in weakness and incoordination; stiff, tight leg muscles; bowel and bladder problems; greater fatigue, depression, and problems of thinking. Although several drugs are known for treating RRMS, SPMS is in general more difficult to treat. Especially anti-inflammatory or immunomodulatory medicaments that are useful for treating RRMS fail in treating SPMS.

Sphingosine-1-phosphate (SIP) receptors belong to a family of closely related, lipid-activated G-protein-coupled receptors. S1P1, S1P3, S1P2, S1P4, and S1P5 (also respectively termed EDG-1, EDG-3, EDG-5, EDG-6 and EDG-8) are identified as receptors specific for SIP. Certain SIP receptors are associated with diseases mediated by lymphocyte interactions, for example, in transplantation rejection, autoimmune disease, inflammatory diseases, infectious diseases and cancer. Hence, SIP receptor modulators are an interesting class of compounds for treating MS.

While SIP receptor modulators are interesting and mostly effective compounds for treating diseases e.g. mediated by lymphocyte interactions, they may produce a negative chronotropic side effect, e.g. at therapeutic doses, i.e. they may reduce the cardiac rhythm (bradycardia), as described in WO 2010/072703 A1. Administration of 10 mg of siponimod may induce a decrease in heart rate of approximately 10 beats/min. While this side effect might not be highly problematic for quite healthy patients, it might be critical for patients with a reduced clinical condition, e.g. patients with SPMS.

As a consequence of this side effect, the SIP modulator therapy has to be initiated under close medical supervision (First Dose Monitoring), generally in a hospital, in order to check that the cardiac rhythm is maintained at an acceptable level.

WO 2012/095853 describes modified release formulations of immunosuppressant compounds, particularly formulations of SIP receptor modulators. In order to minimize the known negative chronotropic side effects and atrioventricular blocks sometimes associated with the administration of some SIP receptor modulators, it suggests providing a zero order, i.e. linear, modified release profile of siponimod, e.g. a zero order release profile for a period of at least 5 hours.

Hence, there is a need for an effective medicament for the treatment of autoimmune diseases, e.g. SPMS, which has reduced side effects, especially one that can prevent bradycardia.

### Summary of the invention

Contrary to the teaching of WO 2012/095853 it has now been surprisingly found that an immediate release dosage form of siponimod can be used in the treatment of an autoimmune disease, preferably for the treatment of SPMS, with significantly reduced or even completely eliminated negative chronotropic side effects, when it is administered to patients who have experienced a specific titration regimen of siponimod.

Thus, the subject of the present invention is siponimod for use in the treatment of an autoimmune disease, wherein an immediate release dosage form comprising 2 mg siponimod is administered once daily to a patient as a maintenance regimen, and wherein the patient has experienced a titration regimen beforehand of 0.25 mg of siponimod at day 1, 0.25 mg at day 2, 0.5 mg at day 3, 0.75 mg at day 4 and 1.25 mg at day 5.

### Brief Description of the Figures

**Figure 1** is a flow diagram for the manufacture of dosage forms comprising siponimod
**Figure 2****:** Use of siponimod to treat relapse and progression in PLP₁₃₉₋₁₅₁ induced EAE in SJL/J mice

### Detailed description of the invention

Surprisingly it has been found that by administering an immediate release dosage form of siponimod to autoimmune disease patients, e.g. SPMS patients, who have experienced a specific titration regimen of siponimod, the negative chronotropic side effects which may be associated with administration of siponimod can be significantly reduced or even completely eliminated.
In particular, an abrupt drop in the heart rate may be avoided. Administering siponimod according to the specific dosage regimen of the present invention may also significantly reduce or even completely eliminate the risk that the patient taking siponimod suffers from heart effects e.g. atrio-ventricular (AV) blocks or heart pauses.
Furthermore the specific dosage regimen of the present invention may facilitate/permit to administer siponimod to categories of patients for which the risk/benefit ratio may otherwise be less favourable.
Such patients could for example include patients suffering from or susceptible to heart problems e.g. heart failure or arrhythmias, patients suffering from or susceptible to high grade atrio-ventricular blocks or sick sinus syndrome, patients with a history of syncopal episodes, or patients undergoing beta blocker or anti-arrhythmic treatment, such as patients under treatment with anti-arrhythmic drugs; or patients that have undergone an interruption or treatment holiday in the siponimod maintenance dosage regime e.g. a holiday of greater than 3 days, greater than 4, 6, 8 or 10 days.
The dosage regimen of the present invention is a regimen for the initiation/maintenance of siponimod therapy, which enables a standard daily therapeutic dosage range of siponimod to be achieved with minimal/no negative chronotropic side effects and/or the AV block effects possibly associated with siponimod therapy.

For the avoidance of doubt, it is hereby stated that the information disclosed earlier in this specification under the heading "Background" is relevant to the invention and is to be read as part of the disclosure of the invention.

Throughout the description and claims of this specification, the words "comprise" and "contain" and variations of them mean "including but not limited to", and they are not intended to (and do not) exclude other moieties, additives, components, integers or steps.

Throughout the description and claims of this specification, the singular encompasses the plural unless the context otherwise requires. In particular, where the indefinite article is used, the specification (which term encompasses both the description and the claims) is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

"Siponimod" as used herein is understood to comprise the compound of formula (I) 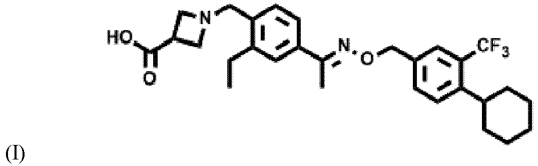 as well as the pharmaceutically acceptable salts, polymorphs, solvates and/or hydrates thereof. Siponimod as used herein has the IUPAC-name 1-{4-[1-((E)-4-cyclohexyl-3-trifluoromethyl-benzyloxyimino)-ethyl]-2-ethyl-benzyl}-azetidine-3-carboxylic acid (BAF312).

In a preferred embodiment of the invention, siponimod is present in the form of siponimod free base or siponimod salt. Examples of pharmaceutically acceptable salts of siponimod include salts with inorganic acids, such as hydrochloride, hydrobromide and sulfate, salts with organic acids, such as acetate, fumarate, hemifumarate, maleate, benzoate, citrate, malate, methanesulfonate and benzenesulfonate salts, or, when appropriate, salts with metals such as sodium, potassium, calcium and aluminium, salts with amines, such as triethylamine and salts with dibasic amino acids, such as lysine. In a preferred embodiment siponimod is in the hemifumarate salt form.

In one embodiment of the invention, siponimod can be provided in an amorphous form or crystalline form, preferably in a crystalline form.

The term "crystalline" can be used in the context of this invention to describe the state of a solid substance, whose constituent atoms, molecules, or ions are arranged in an ordered pattern extending in all three spatial dimensions.

The siponimod of the invention may consist of purely crystalline siponimod. Alternatively, it may also contain small amounts of non-crystalline siponimod components. In an embodiment of the invention the siponimod contained in the inventive dosage form can be 85 to 99.999%, more preferably 90 to 99.99%, most preferably 95 to 99.9% by weight crystalline siponimod.

Generally, siponimod can be used in the dosage form of the present invention in particulate form. The siponimod can preferably have an average particle size X90 of 10 to 100 µm, more preferably 15 to 80 µm, most preferably 30 to 70 µm.

The particle size X90, which is also denoted as X90 value of the integral volume distribution, is defined in the context of this invention as the particle diameter at which 90 per cent by volume of the particles have a smaller diameter than the diameter which corresponds to the X90 value. Likewise, 10 per cent by volume of the particles have a larger diameter than the X90 value. The X10 and X50 values are defined accordingly.

Further, the siponimod can preferably have an average particle size X50 of 1 to 25 µm, more preferably 2 to 22 µm, even more preferably 4 to 20 µm, especially 5 to 17 µm).

Yet further, the siponimod can preferably have a particle size X10 of 0.5 to 5 µm, more preferably 1 to 4 µm, even more preferably 1.2 to 3 µm, especially preferably 1.5 to 2.7 µm.

In a preferred embodiment the ratio X90/X50 can be 1.0 to 100, preferably 1.2 to 10, more preferably 2.5 to 4.0. In a preferred embodiment, the ratio X50/X10 can be 1.1 to 10, preferably 1.2 to 5, more preferably 2.5 to 4.0.

The particle size distribution by volume can be measured using laser diffractometry. In particular, it can be measured using the Sympatec Helos device (from Sympatec GmbH, Germany) using the Cuvette dispersion device. To make the measurement, a stock dispersion was prepared by mixing the drug substance with a dispersing aid (Octastat 5000 (Octel corp)) using a vortex until a smooth and homogeneous paste was formed. The paste was then diluted and mixed to a final volume of 3 to 6 ml using white spirit. The optical concentration of the final solution was kept below 5%. The percentage values were calculated from the mean cumulative volume size curve by the software of the Sympatec instrument. Preferably, the Fraunhofer method is used for calculation purposes.

The siponimod immediate release dosage form of the maintenance regimen contains 2 mg of siponimod, based on the amount of siponimod in form of the free base. Since siponimod can be present in the form of a salt, the amount of the respective salt former (e.g. the respective acid) has to be added accordingly. Similar considerations apply for the dosage forms of the titration regimen. The amounts of 0.25 mg siponimod at day 1, 0.25 mg at day 2, 0.5 mg at day 3, 0.75 mg at day 4 and 1.25 mg at day 5 refer to the amounts of siponimod in free form.

Preferably, an immediate release dosage form is administered in the maintenance regimen as well as in the titration regimen.

Generally, the term "maintenance regime" refers to the administration of siponimod after the up-titration is completed. Said maintenance regime comprises the administration of the maintenance dose of 2 mg. Preferably, the maintenance regime is carried out continuously, for example over several days, weeks, months or years.

Generally, the term "immediate release dosage form" stands for a dosage form with a in-vitro release profile of the dosage form according to USP app. II (paddle, 500 mL for the 0.25 mg dosage strength, 900 mL for the 0.5, 1 and 2 mg dosage strengths, phosphate buffer + 0.1% (m/v) Tween 80, 60 rpm ± 2 rpm, 37°C± 0.5°C) after 30 minutes preferably indicates a content release of at least 80 %, preferably more than 90%, more preferred of more than 95%. The release can be up to 100 %.

Generally, the term "immediate release dosage form" stands for a dosage form with a release profile of the dosage form according to USP app. II (paddle, 900 ml, phosphate buffer + 0.1% (m/v) Tween 80, 60 rpm, 37°C) after 30 minutes preferably indicates a content release of at least 80%, preferably more than 90%, especially more than 95%, or a bioequivalent dosage form to said dosage form.

Tween 80 has the name polyoxyethylene(20) sorbitan monooleate of the formula 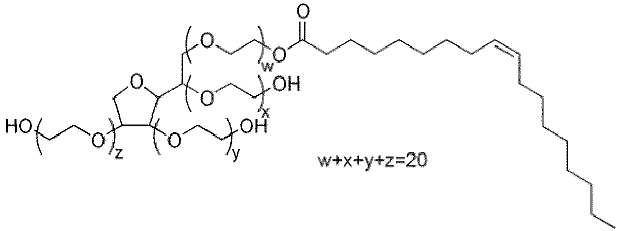 and preferably has a density at 25°C of around 1.06-1.09 g/mL, a viscosity at 25°C of 300-500 mPa·s and a HLB-value (hydrophilic-lipophilic balance value) of 15.0, determined by the Griffin's method.

According to the invention it is understood that a dosage form being a bioequivalent dosage form to a dosage form which is described in Example 1 is an immediate release dosage form.

Generally, the term "bioequivalent dosage form" stands for a dosage form which fulfils the standard of the FDA or EMA with respect to another dosage form, e.g. as described in "FDA Guidance for Industry Bioavailability and Bioequivalence Studies for Orally Administered Drug Products - General Considerations" from 2003 or in "EMEA Note for Guidance on the Investigation of Bioavailability and Bioequivalence Bioequivalence" from 2000. According to these recommendations, the parameters and the corresponding 90% confidence intervals should lie within a range of acceptance of 80 to 125% of the reference preparation. In order to establish bioequivalence, it is necessary for 9 out of 10 of the pharmacokinetic measurement values of the test product to lie within this range of acceptance. Parameters relevant for bioequivalence according to those standards are Cmax and AUClast.

In one embodiment of the invention, the dosage form of siponimod is an oral solid dosage form. In one embodiment of the invention, the dosage form of siponimod is preferably a tablet. Alternatively, the dosage form of the invention could be a capsule.

The immediate release dosage form of the present invention can be formed by providing siponimod as described above and by blending siponimod with at least one pharmaceutical excipient. Typical pharmaceutical excipients comprise lubricants, glidants, fillers, disintegrants, moisture protecting agents and binders.

Fillers generally are substances suitable to add volume and/or mass to a drug substance, thereby facilitating precise metering and handling thereof in the preparation of dosage forms. Fillers typically also fill out the size of a tablet or capsule, making it practical to produce and convenient for the consumer to use.

Suitable fillers are plant cellulose (pure plant filler), hydroxypropyl cellulose, calcium phosphate, calcium hydrogen phosphate, calcium dihydrogen phosphate, calcium carbonate, magnesium carbonate, magnesium aluminosilicates, sugar alcohols, such as mannitol, maltitol, isomalt, sorbitol, xylitol, threitol and erythritol, a triglyceride, such as hydrogenated vegetable oil, mucilage such as carrageenan, agar and pectin, a monosaccharide such as arabinose, xylose, glucose, mannose, galactose, a disaccharide, such as isomaltose, maltose, lactose, sucrose, an oligosaccharide, such as raffinose, oligofructose, cyclodextrins, maltodextrin, a polysaccharide, such as starch, such as corn starch, glycogen and cellulose, such as microcrystalline cellulose, and mixtures thereof. Preferably, microcrystalline cellulose and lactose can be used as fillers.

Generally, the dosage form of the present invention can comprise 0 to 90 wt.-% fillers, preferably 20 to 90 wt.-%, more preferably 30 to 80 wt.-%, based on the total weight of the dosage form.

Lubricants are generally substances suitable to reduce sliding friction. In particular, the intention is to reduce the sliding friction found during tablet pressing between the punch moving up and down in the die and the die wall, on the one hand, and between the edge of the tablet and the die wall, on the other hand. Suitable lubricants are, for example, stearic acid, adipic acid, sodium stearyl fumarate and/or glyceryl behenate.

Generally, the dosage form of the present invention can comprise 0 to 10 wt.-% lubricants, preferably 0.01 to 8 wt.-%, more preferably 0.1 to 5 wt.-%, based on the total weight of the dosage form.

Binders are substances that ensure that granules or tablets can be formed with the required mechanical strength. Binders can be, for example, saccharose, gelatine, polyvinylpyrrolidone, starch, cellulose derivatives such as hydroxylpropyl cellulose, hydroxyethyl cellulose, hydroxypropyl methylcellulose (HPMC). Preferably, polyvinylpyrrolidone can be used as binder.

Generally, the dosage form of the present invention can comprise 0 to 30 wt.-% binders, preferably 1 to 15 wt.-%, more preferably 2 to 10 wt.-%, based on the total weight of the dosage form.

Glidants can be used to improve the flowability. A preferred glidant is colloidal silica.

Generally, the dosage form of the present invention can comprise 0 to 10 wt.-% glidants, preferably 0.1 to 5 wt.-%, more preferably 1 to 3 wt.-%, based on the total weight of the dosage form.

Disintegrants are substances which can enhance the ability of the intermediate to break into smaller fragments when in contact with a liquid, preferably water. Preferred disintegrants are guar galactomannan, sodium carboxymethyl starch (croscarmellose sodium), cross-linked polyvinylpyrrolidone (crospovidone), sodium carboxymethyl glycolate, sodium bicarbonate or mixtures thereof. Preferably croscarmellose and crospovidone can be used.

Generally, the dosage form of the present invention can comprise 0 to 20 wt.-% disintegrants, preferably 1 to 12 wt.%, more preferably 2 to 8 wt.-%, based on the total weight of the dosage form.

In a preferred embodiment of the invention, the immediate release dosage form comprises a moisture protective agent. The moisture protective agent is a substance suitable to protect the siponimod particles from moisture during the dosage form formation process and/or storage.

In one embodiment, the moisture protective agent is selected from hydrogenated vegetable oil, castor oil, palmitol stearate, glyceryl palmitostearate and glyceryl behenate. Preferably, glyceryl behenate is used as moisture protective agent. The moisture protective agent can also have lubricating properties. In case a moisture protective agent is used it is preferred that the dosage form does not contain any additional lubricants.

Generally, the dosage form of the present invention can comprise 0 to 20 wt.-% moisture protective agent, preferably 0.1 to 12 wt.-%, more preferably 1 to 8 wt.-%, based on the total weight of the dosage form.

In a preferred embodiment, a dosage form of the present invention comprises
0.1 to 10 wt.%, preferably 0.2 to 5 wt. % siponimod,
0 to 10 wt.%, preferably 0.2 to 6 wt. % moisture protective agent,
0 to 15 wt.%, preferably 1 to 8 wt. % disintegrant,
0 to 15 wt.%, optionally 1 to 8 wt. % binder,
0 to 99.9 wt.%, preferably 50 to 90 wt. % filler, and
0 to 10 wt.%, preferably 0.2 to 5 wt. % glidant.

In a preferred embodiment, a dosage form of the present invention, preferably for use in the maintenance regimen comprises
2 mg siponimod,
0 to 15 mg, preferably 1 to 8 mg moisture protective agent,
0 to 25 mg, preferably 0.5 to 15 mg disintegrant,
15 to 250 mg, preferably 30 to 85 mg filler,
0 to 50 mg, optionally 5 to 20 mg binder, and
0 to 20 mg glidant, preferably 1 to 10 mg glidant.

In a preferred embodiment, a dosage form of the present invention, preferably for use in the titration regimen comprises
0.25 mg, 0.5 mg, 0.75 mg or 1 mg siponimod,
0 to 15 mg, preferably 1 to 8 mg moisture protective agent,
0 to 25 mg, preferably 0.5 to 15 mg disintegrant,
15 to 250 mg, preferably 30 to 85 mg filler,
0 to 50 mg, optionally 5 to 20 mg binder, and
0 to 20 mg glidant, preferably 1 to 10 mg glidant.

The above-mentioned mixtures of siponimod with the at least one pharmaceutical excipient can be transformed into a dosage form, e.g. a capsule or a tablet, preferably a tablet.

The dosage form can be formed by direct compression. Hence, the above blending mixtures can be compressed into tablets.

Alternatively, the dosage form can be formed by dry granulation. Dry granulation involves the steps of dry powder blending, initial compaction (slugging or roller compaction), milling, addition of extragranular excipients and lubrication before compaction or capsule filling.

Further, if the dosage form is a tablet, the tablet can be film-coated. For this purpose, standard methods of film coating tablets may be employed. The above-mentioned amounts of siponimod and excipients, however, relate to the uncoated tablet.

For film coating, macromolecular substances are preferably used, such as modified celluloses, polymethacrylates, polyvinylpyrrolidone, polyvinyl acetate phthalate and/or shellac. In an embodiment the coating can have a thickness of 2 to 80 µm, more preferably 5 to 50 µm.

The preferred dosage forms have been described above. In a further preferred embodiment, the 2 mg dosage form of the maintenance regimen is such that the administration of a single dosage form leads in-vivo to a Cmax of 10 to 20 ng/ml, preferably 14.0 to 17.0 ng/ml, more preferably 14.5 to 16.5 ng/ml, still more preferably 15.0 to 16.0 ng/ml, and to a AUClast of 300 to 700 h·ng/ml, preferably 500 to 560 h·ng/ml, more preferably 510 to 550 h·ng/ml, still more preferably 520 to 540 h·ng/ml.

"Cmax" means the peak concentration of siponimod in the plasma, e.g. determined as described below. "AUClast" describes siponimod bioavailability and is measured by calculating the area under curve (AUC) of the plasma drug concentration time profile from time zero to the time of the last quantifiable concentration. AUClast can be determined as described below.

In a further preferred embodiment, the in-vivo AUC-time profile from time zero to infinity (*AUCinf*) of a single 2 mg dosage form of the maintenance regimen is 350 to 750 h·ng/ml, preferably 520 to 600 h·ng/ml, more preferably 540 to 580 h·ng/ml, still more preferably 550 to 570 h·ng/ml.

In a further preferred embodiment of the invention, the administration of a single 2 mg siponimod dosage form of the maintenance regimen leads in-vivo to a Tmax of 3 to 8 h, preferably3 to 7 h, more preferably 3 to 6 h, most preferably 3 to 5 h.

"Tmax" means the time from administration to reach Cmax.

The Cmax, AUClast, AUCinf and Tmax values typically can be determined in vivo in healthy subjects, aged 20 to 40 years. The subjects typically have maximal ±10% divergence from the ideal weight, in order to minimize a strong fluctuation of the distribution volume. Ideally, the administration of the medicament occurs on an empty stomach. The type and amount of the administration liquid is identical for every administration and for every subject. Blood samples are taken at the initial phase with a higher frequency than at a later stage, in order to increase the accuracy of the measurement.
The AUC values are calculated after one administration, preferably using the Trapezoidal Rule.

In a further preferred embodiment, the 0.25 mg dosage form of the titration regimen is such that the administration of a single dosage form leads in-vivo to a Cmax of 1.3 to 2.6 ng/ml, preferably 1.5 to 2.4 ng/ml, more preferably 1.7 to 2.2 ng/ml, still more preferably 1.9 to 2.0 ng/ml, and to a AUClast of 45 to 90 h·ng/ml, preferably 50 to 80 h·ng/ml, more preferably 55 to 75 h·ng/ml, still more preferably 60 to 70 h·ng/ml.
In a further preferred embodiment, the in-vivo AUC-time profile from time zero to infinity (*AUCinf*) of a single 0.25 mg dosage form of the titration regimen is 47 to 95 h·ng/ml, preferably 55 to 85 h·ng/ml, more preferably 60 to 80 h·ng/ml, still more preferably 65 to 75 h·ng/ml.

In a further preferred embodiment of the invention, the administration of a single 0.25 mg siponimod dosage form of the titration regimen leads in-vivo to a Tmax of 3 to 8 h, preferably3 to 7 h, more preferably 3 to 6 h, most preferably 3 to 5 h.

In a further preferred embodiment, the 0.5 mg dosage form of the titration regimen is such that the administration of a single dosage form leads in-vivo to a Cmax of 2.6 to 5.2 ng/ml, preferably 3.0 to 4.8 ng/ml, more preferably 3.4 to 4.4 ng/ml, still more preferably 3.7 to 4.0 ng/ml, and to a AUClast of 90 to 180 h·ng/ml, preferably 100 to 160 h·ng/ml, more preferably 110 to 150 h·ng/ml, still more preferably 120 to 140 h·ng/ml.
In a further preferred embodiment, the in-vivo AUC-time profile from time zero to infinity (*AUCinf*) of a single 0.5 mg dosage form of the titration regimen is 95 to 190 h·ng/ml, preferably 110 to 170 h·ng/ml, more preferably 120 to 160 h·ng/ml, still more preferably 130 to 150 h·ng/ml.
In a further preferred embodiment of the invention, the administration of a single 0.5 mg siponimod dosage form of the titration regimen leads in-vivo to a Tmax of 3 to 8 h, preferably3 to 7 h, more preferably 3 to 6 h, most preferably 3 to 5 h.

In a further preferred embodiment, the 1 mg dosage form of the titration regimen is such that the administration of a single dosage form leads in-vivo to a Cmax of 5 to 10 ng/ml, preferably 5.5 to 9.5 ng/ml, more preferably 6 to 9 ng/ml, still more preferably 7 to 8 ng/ml, and to a AUClast of 180 to 360 h·ng/ml, preferably 200 to 320 h·ng/ml, more preferably 220 to 300 h·ng/ml, still more preferably 240 to 280 h·ng/ml.
In a further preferred embodiment, the in-vivo AUC-time profile from time zero to infinity (*AUCinf*) of a single 1 mg dosage form of the titration regimen is 190 to 370 h·ng/ml, preferably 220 to 340 h·ng/ml, more preferably 140 to 320 h·ng/ml, still more preferably 260 to 300 h·ng/ml.
In a further preferred embodiment of the invention, the administration of a single 1 mg siponimod dosage form of the titration regimen leads in-vivo to a Tmax of 3 to 8 h, preferably3 to 7 h, more preferably 3 to 6 h, most preferably 3 to 5 h.

In one embodiment, the siponimod of the present invention is administered once daily in the maintenance regimen as well as in the titration regimen the patient has experienced beforehand.
In one embodiment, the patient has experienced a titration regimen beforehand wherein dosage forms comprising 0.25 mg, 0.5 mg or 1 mg siponimod have been used.
In one embodiment, the patient has experienced a titration regimen beforehand of one administration of a dosage form comprising 0.25 mg siponimod at day 1, one administration of a dosage form comprising 0.25 mg siponimod at day 2, one administration of a dosage form comprising 0.5 mg siponimod at day 3, one concomitant administration of a dosage form comprising 0.5 mg siponimod together with a dosage form comprising 0.25 mg siponimod at day 4, and one concomitant administration of a dosage form comprising 1 mg siponimod together with a dosage form comprising 0.25 mg siponimod at day 5.

The siponimod of the present invention is used in the treatment of an autoimmune disease, such as multiple sclerosis (MS), for example relapsing-remitting MS (RRMS), primary progressive MS (PPMS), secondary progressive MS (SPMS) and relapsing SPMS. The siponimod of the present invention is preferably used for the treatment of RRMS and/or SPMS, most preferably SPMS.

"SPMS" is defined as "initial relapsing remitting disease course followed by progression with or without occasional relapses, minor remissions, and plateaus" (Lublin, F.D., Reingold, S.C. (1996) Defining the clinical course of multiple sclerosis. Neurology, 46: 907-911). The diagnosis of MS with initial relapsing remitting disease course is defined by the 2010 Revised McDonald criteria (Polman CH, Reingold S, Banwell B, et al. (2011). Diagnostic criteria for multiple sclerosis: 2010 revisions to the McDonald criteria. Ann Neurol; 68: 292-302). Progression denotes the continuous worsening of neurological impairment over at last 6 months (Rovaris M., Confavreux C., Furlan R. et al. (2006). Secondary progressive multiple sclerosis: current knowledge and future challenges; Lancet Neurology 5: 343-354) not explained by incomplete recovery from relapses (Lublin, F.D., Baier, M., Cutter, G. (2003) Effect of relapses on development on residual deficit in multiple sclerosis. Neurology, 51: 1528-1532).

In one embodiment, siponimod is used for the treatment of SPMS patients characterized by a progressive increase in disability of at least 6 months' duration in the absence of relapses or independent of relapses.

In one embodiment, siponimod is used for the treatment of SPMS patients having a disability status with an EDSS score of 2.0 to 8.0, more preferably 2.5 to 7.0, most preferably 3.0 to 6.5.

"EDSS" stands for the Kurtzke Expanded Disability Status Scale, which is a method of quantifying disability in multiple sclerosis (cf. Table 1). The EDSS quantifies disability in eight Functional Systems (FS) and allows neurologists to assign a Functional System Score (FSS) in each of these. Kurtzke defines functional systems as follows: pyramidal, cerebellar, brainstem, sensory, bowel and bladder, visual, cerebral, other.

The Functional Systems (FS) are scored on a scale of 0 (low level of problems) to 5 (high level of problems) to best reflect the level of disability observed clinically. The "Other" category is not rated numerically, but measures disability related to a particular issue, like motor loss.

In contrast, the total EDSS score is determined by two factors: gait and FS scores. EDSS scores below 4.0 are determined by the FS scores alone. People with EDSS scores of 4.0 and above have some degree of gait impairment. Scores between 4.0 and 9.5 are determined by both gait abilities and the FS scores.

**Table 1 - Kurtzke Expanded Disability Status Scale**

| | |
|---|---|
| 0 | Normal neurological exam (all grade 0 in Functional Systems (FS); cerebral grade 1 acceptable). |
| 1 | No disability, minimal signs in one FS (i.e. one grade 1 excluding cerebral grade 1). |
| 1.5 | No disability, minimal signs in more than one FS (more than one grade 1 excluding cerebral grade 1). |
| 2.0 | Minimal disability in one FS (one FS grade 2, others 0 or 1). |
| 2.5 | Minimal disability in two FS (two FS grade 2, others 0 or 1). |
| 3.0 | Moderate disability in one FS (one FS grade 3, others 0 or 1), or mild disability in three or four FS (three-four FS grade 2, others 0 or 1). |
| 3.5 | Fully ambulatory but with moderate disability in one FS (one grade 3 and one or two FS grade 2) or two FS grade 3, others 0 or 1, or five FS grade 2, others 0 or 1. |
| 4.0 | Fully ambulatory without aid, self-sufficient, up and about some 12 hours a day despite relatively severe disability consisting of one FS grade 4 (others 0 or 1), or combinations of lesser grades exceeding limits of previous steps. Able to walk without aid or rest some 500 meters (0.3 miles). |
| 4.5 | Fully ambulatory without aid, up and about much of the day, able to work a full day, may otherwise have some limitation of full activity or require minimal assistance; characterized by relatively severe disability. (Usually consisting of one FS grade 4 (others 0 or 1) or combinations of lesser grades exceeding limits of previous steps. Able to walk without aid or rest for some 300 meters (975 ft.).) |
| 5.0 | Ambulatory without aid or rest for about 200 meters (650 ft.); disability severe enough to impair full daily activities (e.g. to work full day without special provisions). (Usual FS equivalents are one grade 5 alone (others 0 or 1); or combinations of lesser grades usually exceeding specifications for step 4.0.) |
| 5.5 | Ambulatory without aid or rest for about 100 meters (325 ft); disability severe enough to impair full daily activities. (Usual FS equivalents are one grade 5 alone (others 0 or 1); or combinations of lesser grades usually exceeding specifications for step 4.0.) |
| 6.0 | Intermittent or constant unilateral assistance (cane, crutch or brace) required to walk about 100 meters (325 ft.) with or without resting. (Usual FS equivalents are combinations with more than two FS grade 3+.) |
| 6.5 | Constant bilateral assistance (canes, crutches or braces) required to walk about 20 meters (65 ft.). (Usual FS equivalents are combinations with more than two FS grade 3+.) |
| 7.0 | Unable to walk beyond about 5 meters (16 ft.) event with aid, essentially restricted to wheelchair, wheels self in standard wheelchair a full day and transfers alone; up and about in wheelchair some 12 hours a day. (Usual FS equivalents are combinations with more than one FS grade 4+; very rarely pyramidal grade 5 alone.) |
| 7.5 | Unable to take more than a few steps; restricted to wheelchair; may need aid in transfers, wheels self but cannot carry on in standard wheelchair a full day; may require motorized wheelchair. (Usual FS equivalents are combinations with more than one FS grade 4+.) |
| 8.0 | Essentially restricted to bed or chair or perambulated in wheelchair; but may be out of bed much of the day; retains many self-care functions; generally has effective use of arms. (Usual FS equivalents are combinations, generally grade 4+ in several systems.) |
| 8.5 | Essentially restricted to bed for much of the day; has some effective use of arm(s); retains some self-care functions. (Usual FS equivalents are combinations, generally grade 4+ in several systems.) |
| 9.0 | Helpless bed patient; can communicate and eat. (Usual FS equivalents are combinations, mostly grade 4.) |
| 9.5 | Totally helpless bed patient; unable to communicate or effectively eat/swallow. (Usual FS equivalents are combinations, almost all grade 4+.) |
| 10 | Death due to MS. |

The terms "treatment"/"treating" as used herein includes: (1) preventing or delaying the appearance of clinical symptoms of the state, disorder or condition developing in an animal, particularly a mammal and especially a human, that may be afflicted with or predisposed to the state, disorder or condition but does not yet experience or display clinical or subclinical symptoms of the state, disorder or condition; (2) inhibiting the state, disorder or condition (e.g. arresting, reducing or delaying the development of the disease, or a relapse thereof in case of maintenance treatment, of at least one clinical or subclinical symptom thereof); and/or (3) relieving the condition (i.e. causing regression of the state, disorder or condition or at least one of its clinical or subclinical symptoms). The benefit to a patient to be treated is either statistically significant or at least perceptible to the patient or to the physician. However, it will be appreciated that when a medicament is administered to a patient to treat a disease, the outcome may not always be effective treatment.

In one embodiment, the siponimod of the present invention is effective to reduce a symptom of RRMS or SPMS, preferably SPMS. In one embodiment, the symptom is an MRI-monitored multiple sclerosis disease activity, disability progression, brain atrophy, neuronal dysfunction, neuronal injury, neuronal degeneration, deterioration of visual function, impaired mobility, cognitive impairment, reduction of brain volume, deterioration in general health status, functional status and/or quality of life.

In one embodiment, the siponimod of the present invention, in patients with SPMS, delays disability progression, e.g. as assessed by EDSS.
In one embodiment, the treatment comprises increasing the time to 3-month confirmed disability progression in patients with SPMS as measured by EDSS compared to untreated patients. Disability progression as measured by EDSS typically is defined as an increase from the baseline of at least 1 point (in patients with a baseline EDSS score of 3.0 to 5.0) or of at least 0.5 point (in patients with a baseline EDSS score of 5.5. to 6.5). To confirm that the progression is sustained, this increase must be present at a visit 3 months later.
In one embodiment, time to 3-month confirmed disability progression is increased by at least 10%.
In one embodiment, time to 3-month confirmed disability progression is increased by at least 25%.

In one embodiment, time to 3-month confirmed disability progression is increased by 20 to 75%.
In one embodiment, time to 3-month confirmed disability progression is increased by 10 to 75%.
In another embodiment, time to 3-month confirmed disability progression is increased by 25 to 50%.
In another embodiment, time to 3-month confirmed disability progression is increased by 25 to 40%.

In one embodiment, the siponimod of the present invention, in patients with SPMS, delays worsening of impaired mobility, e.g. as assessed by the timed 25-foot walk test (T25-FW).
The T25-FW is a quantitative mobility and leg function performance test based on a timed 25-walk. The patient is directed to one end of a clearly marked 25-foot course and is instructed to walk 25 feet as quickly as possible, but safely. The time is calculated from the initiation of the instruction to start and ends when the patient has reached the 25-foot mark. The task is immediately repeated again by having the patient walk back the same distance. The T25-FW is one of three components of the Multiple Sclerosis Functional Composite (MSFC), a composite measure assessing upper extremity function, ambulation and cognitive function (Fisher JS et al. for the National MS Society Clinical Outcomes Assessment Task Force (1999). The multiple sclerosis functional composite measure: an integrated approach to MS clinical outcome assessment. Mult Scler; 5: 244-250).
In one embodiment, treatment comprises delaying the time to 3-month confirmed worsening of at least 20% from the baseline in the T25-FW compared to untreated patients.
In one embodiment, time to 3-month confirmed worsening of at least 20% from the baseline in the T25-FW compared to untreated patients is increased by at least 10%.
In one embodiment, time to 3-month confirmed worsening of at least 20% from the baseline in the T25-FW compared to untreated patients is increased by at least 25%.
In one embodiment, time to 3-month confirmed worsening of at least 20% from the baseline in the T25-FW compared to untreated patients is increased by 10 to 80%.
In one embodiment, time to 3-month confirmed worsening of at least 20% from the baseline in the T25-FW compared to untreated patients is increased by 20 to 80%.
In another embodiment, time to 3-month confirmed worsening of at least 20% from the baseline in the T25-FW compared to untreated patients is increased by 25 to 70%.
In another embodiment, time to 3-month confirmed worsening of at least 20% from the baseline in the T25-FW compared to untreated patients is increased by 25 to 50%.

In one embodiment, the siponimod of the present invention, in patients with SPMS, reduces the increase in T2 lesion volume, e.g. increase within 2 years of treatment from the baseline compared to untreated patients, as measured by Magnetic Resonance Imaging (MRI). T2 lesions are detected using MR-images that emphasize T2 contrast. T2 lesions represent new inflammatory activity.
In one embodiment, increase in T2 lesion volume within 2 years of treatment from the baseline is reduced compared to untreated patients by at least 10%.
In one embodiment, increase in T2 lesion volume within 2 years of treatment from the baseline is reduced compared to untreated patients by at least 25%.

In one embodiment, increase in T2 lesion volume within 2 years of treatment from the baseline is reduced compared to untreated patients by 10 to 100%.
In one embodiment, increase in T2 lesion volume within 2 years of treatment from the baseline is reduced compared to untreated patients by 20 to 100%.
In another embodiment, increase in T2 lesion volume within 2 years of treatment from the baseline is reduced compared to untreated patients by 25 to 90%.
In another embodiment, increase in T2 lesion volume within 2 years of treatment from the baseline is reduced compared to untreated patients by 30 to 80%.

In one embodiment, the siponimod of the present invention decreases or inhibits reduction of brain volume in patients with SPMS, measured by percent brain volume change.

In one embodiment, the siponimod of the present invention, in patients with SPMS, increases a general health status or delays a deterioration of the general health status, as defined by the EQ-5D. EQ-5D is a standardized questionnaire used for measuring the general health status. It measures five domains (mobility, self-care, usual activity, pain/discomfort, anxiety/depression).

In one embodiment, the siponimod of the present invention, in patients with SPMS, increases or delays a reduction of the health-related quality of life as measured by the Multiple Sclerosis Impact Scale (MSIS-29), an instrument measuring the physical (20 items) and psychological (nine items) impact of multiple sclerosis.

According to the invention, the immediate release dosage form comprising 2 mg siponimod is administered once daily to a patient as a maintenance regimen, wherein the patient has experienced a titration regimen beforehand of 0.25 mg siponimod at day 1, 0.25 mg at day 2, 0.5 mg at day 3, 0.75 mg at day 4 and 1.25 mg at day 5. It has been found that administering siponimod to a patient according to said titration regimen reduces the risk of negative chronotropic side effects, especially bradycardia, when the 2 mg siponimod maintenance regimen is then administered. Hence, the treatment with 2 mg siponimod does not have to be initiated under close medical supervision (First Dose Monitoring). Consequently, this has beneficial effects for the patient, as the treatment does not need to be started in a hospital for monitoring and the health risks are minimized.

In one embodiment, no negative chronotropic side effect, especially no reduction of heart beat, is observed after administration of the 2 mg siponimod immediate release dosage form (maintenance dose) after the 5 days titration.

The siponimod used in the titration regimen can be identical with or different from the siponimod used in the 2 mg immediate release dosage form. Preferably, the siponimod used in the titration regimen is identical with the siponimod used in the 2 mg immediate release dosage form.

In a preferred embodiment of the invention, the siponimod used in the titration regimen and in the 2 mg immediate release dosage form is siponimod hemifumarate. Generally, all explanations given above of preferred embodiments of siponimod (e.g. salts, particle size), excipients and dissolution behaviour preferably apply to the dosage form of the maintenance regimen as well as for the dosage form of the titration regimen.

A further aspect of the invention is a method of treating a patient with an autoimmune condition.

The method of treating comprises administering an initial titration regimen of siponimod, and administering an immediate release dosage form of 2 mg siponimod as a maintenance regimen, wherein the titration regimen comprises administering 0.25 mg siponimod at day 1, 0.25 mg of siponimod at day 2,0.5 mg of siponimod at day 3,0.75 mg of siponimod at day 4 and 1.25 mg of siponimod at day 5.

An example of an autoimmune condition according to the present invention is multiple sclerosis (MS), for example relapsing-remitting MS (RRMS), primary progressive MS (PPMS), secondary progressive MS (SPMS) and relapsing SPMS. Preferably, the siponimod of the present invention is used for treating RRMS and/or SPMS, most preferably SPMS.

A further aspect of the invention is a kit containing daily units of medication of siponimod of 0.25 mg, 0.25 mg, 0.5 mg, 0.75 mg and 1.25 mg, wherein the kit contains a dosage form comprising 0.25 mg siponimod. The kit may also contain instructions for use.

In an embodiment, the kit may comprise just one dosage form comprising 0.25 mg siponimod. A patient may then take one of said dosage forms on day 1 and day 2, two dosage forms on day 3, three dosage forms on day 4 and five dosage forms on day 5; on day 6, the patient starts the maintenance regimen.

In an alternative embodiment, the kit may comprise a number of low-dose siponimod dosage forms wherein their dosages are compatible for use in the titration regimen of the invention. For example, the kit may comprise two, three or four e.g. three different low-dose siponimod dosage forms. A "low-dose siponimod dosage form" is a form comprising no more than 1.25 mg siponimod.

In an aspect, the kit may comprise a pack, e.g. a pack containing one to five, e.g. two to four, e.g. three or four different dosage forms. The pack may comprise individual storage portions, each portion containing the patient's daily dosage for a given day during the course of treatment. The daily dosage may be made up of one or more of the different dosage forms. In an aspect of this embodiment, the kit comprises a blister pack containing two to four, e.g. three different dosage forms, in which the blisters in the pack contain the daily dosages for administration to the patient during the titration regimen, wherein the daily dosage is made up of one or more of the different dosage forms. In an aspect of this embodiment, the pack, e.g. the blister pack, may comprise a number of blisters corresponding to the number of days of the initial treatment period. In another aspect, the blister pack may also contain one or more blisters containing the therapeutic dose of the maintenance regimen, i.e. 2 mg siponimod, so that the total treatment period including the low dosage and therapeutic dosage form lasts for a clinically convenient period of time, e.g. one week or two weeks.

A further aspect of the invention is a kit containing daily units of medication of siponimod of 0.25 mg, 0.25 mg, 0.5 mg, 0.75 mg and 1.25 mg, wherein the kit contains an immediate release dosage form comprising 0.25 mg siponimod, an immediate release dosage form comprising 0.5 mg siponimod and an immediate release dosage form comprising 1 mg siponimod for administration to a patient according to a titration regimen of 0.25 mg siponimod at day 1, 0.25 mg at day 2, 0.5 mg at day 3, 0.75 mg at day 4 and 1.25 mg at day 5.

A further aspect of the invention is a kit containing daily units of medication of siponimod of 0.25 mg, 0.25 mg, 0.5 mg, 0.75 mg and 1.25 mg, wherein the kit contains an immediate release dosage form comprising 0.25 mg siponimod, an immediate release dosage form comprising 0.5 mg siponimod and an immediate release dosage form comprising 1 mg siponimod for administration to a patient according to a titration regimen of 0.25 mg siponimod at day 1, 0.25 mg at day 2, 0.5 mg at day 3, 0.75 mg at day 4 and 1.25 mg at day 5 and wherein after the titration regimen an immediate release dosage form comprising 2 mg siponimod is administered once daily to said patient as a maintenance regimen.

Further aspects of the invention are:
Embodiment 1: Siponimod for use in the treatment of an autoimmune disease,
   wherein an immediate release dosage form comprising 2 mg siponimod is administered once daily to a patient as a maintenance regimen, and
   wherein the patient has experienced a titration regimen beforehand of 0.25 mg siponimod at day 1, 0.25 mg at day 2, 0.5 mg at day 3, 0.75 mg at day 4 and 1.25 mg at day 5.
Embodiment 2: Siponimod for use according to embodiment 1, wherein the autoimmune disease is Secondary Progressive Multiple Sclerosis.
Embodiment 3: Siponimod for use in the treatment of Secondary Progressive Multiple Sclerosis according to embodiment 2, wherein the patient has experienced a titration regimen beforehand of one administration of 0.25 mg siponimod at day 1, one administration of 0.25 mg siponimod at day 2, one administration of 0.5 mg siponimod at day 3, one administration of 0.75 mg siponimod at day 4, and one administration of 1.25 mg siponimod at day 5.
Embodiment 4: Siponimod for use in the treatment of Secondary Progressive Multiple Sclerosis according to embodiment 3, wherein, in the titration regimen, immediate release dosage forms comprising 0.25 mg, 0.5 mg and 1 mg siponimod have been used.
Embodiment 5: Siponimod for use in the treatment of Secondary Progressive Multiple Sclerosis according to embodiment 2, wherein the immediate release dosage form shows an in-vitro release profile of siponimod of at least 80% after 30 minutes, measured according to USP app. II paddle, 900 ml, phosphate buffer and 0.1% (m/v) Tween 80, 60 rpm, 37°C.
Embodiment 6: Siponimod for use in the treatment of Secondary Progressive Multiple Sclerosis according to embodiment 4,
   wherein the immediate release dosage forms comprising 0.5 mg, 1 mg and 2 mg siponimod show an in-vitro release profile of siponimod of at least 80% after 30 minutes, measured according to USP app. II paddle, 500 ml, phosphate buffer and 0.1% (m/v) Tween 80, 60 rpm, 37°C; and
   wherein the immediate release dosage form comprising 0.25 mg siponimod show an in-vitro release profile of siponimod of at least 80% after 30 minutes, measured according to USP app. II paddle, 900 ml, phosphate buffer and 0.1% (m/v) Tween 80, 60 rpm, 37°C.
Embodiment 7: Siponimod for use in the treatment of Secondary Progressive Multiple Sclerosis according to embodiment 2, wherein the immediate release dosage form comprising 2 mg siponimod is such that the administration of a single dosage form leads in-vivo to a Cmax of 14.0 to 17.0 ng/ml and to a AUClast of 500 to 560 h·ng/ml.
Embodiment 8: Siponimod for use in the treatment of Secondary Progressive Multiple Sclerosis according to embodiment 4,
   wherein the immediate release dosage form comprising 2 mg siponimod is such that the administration of a single dosage form leads in-vivo to a Cmax of 14.0 to 17.0 ng/ml and to a AUClast of 500 to 560 h·ng/ml;
   wherein the immediate release dosage form comprising 1 mg siponimod is such that the administration of a single dosage form leads in-vivo to a Cmax of 5.5 to 9.5 ng/ml and to a AUClast of 200 to 320 h·ng/ml; wherein the immediate release dosage form comprising 0.5 mg siponimod is such that the administration of a single dosage form leads in-vivo to a Cmax of 3.0 to 4.8 ng/ml and to a AUClast of 100 to 160 h·ng/ml; and
   wherein the immediate release dosage form comprising 0.25 mg siponimod is such that the administration of a single dosage form leads in-vivo to a Cmax of 1.5 to 2.4 ng/ml and to a AUClast of 50 to 80 h·ng/ml.
Embodiment 9: Siponimod for use in the treatment of Secondary Progressive Multiple Sclerosis according to any one of embodiments 2 to 8, wherein the dosage form of the maintenance regimen is a tablet comprising
   - 2 mg siponimod,
   - 0.5 to 10 mg moisture protective agent,
   - 0 to 25 mg, preferably 0.5 to 15 mg disintegrant,
   - 15 to 200 mg, preferably 30 to 85 mg filler.
Embodiment 10: Siponimod for use in the treatment of Secondary Progressive Multiple Sclerosis according to embodiment 9, wherein the moisture protective agent is selected from hydrogenated vegetable oil, castor oil, palmitol stearate, glyceryl palmitostearate and glyceryl behenate.
Embodiment 11: Siponimod for use in the treatment of Secondary Progressive Multiple Sclerosis according to any one of embodiments 2 to 9, wherein the patients have EDSS scores of 3.0 to 6.5.
Embodiment 12: Siponimod for use in the treatment of Secondary Progressive Multiple Sclerosis by delaying disability progression,
   wherein an immediate release dosage form comprising 2 mg siponimod is administered once daily to a patient as a maintenance regimen, and
   wherein the patient has experienced a titration regimen beforehand of 0.25 mg siponimod at day 1, 0.25 mg at day 2, 0.5 mg at day 3, 0.75 mg at day 4 and 1.25 mg at day 5.
Embodiment 13: Siponimod for use in the treatment of Secondary Progressive Multiple Sclerosis by delaying disability progression according to embodiment 12, wherein the disability progression is measured as time to 3-month confirmed disability progression by EDSS compared to untreated patients, and wherein the time is increased by 10 to 75%.
Embodiment 14: Siponimod for use in the treatment of Secondary Progressive Multiple Sclerosis by delaying the worsening of impaired mobility,
   wherein an immediate release dosage form comprising 2 mg siponimod is administered once daily to a patient as a maintenance regimen, and
   wherein the patient has experienced a titration regimen beforehand of 0.25 mg siponimod at day 1, 0.25 mg at day 2, 0.5 mg at day 3, 0.75 mg at day 4 and 1.25 mg at day 5.
Embodiment 15: Siponimod for use in the treatment of Secondary Progressive Multiple Sclerosis by delaying the worsening of impaired mobility according to embodiment 14, wherein the worsening of impaired mobility is measured as time to 3-month confirmed worsening of impaired mobility of at least 20% from the baseline in the timed 25-foot walk test, and wherein the time is increased by 10 to 80%.
Embodiment 16: Siponimod for use in the treatment of Secondary Progressive Multiple Sclerosis by reducing the increase in T2 lesion volume,
   wherein an immediate release dosage form comprising 2 mg siponimod is administered once daily to a patient as a maintenance regimen, and
   wherein the patient has experienced a titration regimen beforehand of 0.25 mg siponimod at day 1, 0.25 mg at day 2, 0.5 mg at day 3, 0.75 mg at day 4 and 1.25 mg at day 5.
Embodiment 17: Siponimod for use in the treatment of Secondary Progressive Multiple Sclerosis by reducing the increase in T2 lesion volume according to embodiment 16, wherein the increase in T2 lesion volume is measured within 2 years of treatment, and wherein the increase is reduced by 10 to 100%.
Embodiment 18: A method of treating a patient with an autoimmune condition comprising administering an immediate release dosage form comprising 2 mg siponimod once daily to said patient as a maintenance regimen, and
   wherein said patient has experienced a titration regimen beforehand of 0.25 mg siponimod at day 1, 0.25 mg at day 2, 0.5 mg at day 3, 0.75 mg at day 4 and 1.25 mg at day 5.
Embodiment 19: A method of treating a patient with an autoimmune condition comprising administering an initial titration regimen of siponimod, and administering an immediate release dosage form comprising 2 mg siponimod once daily to said patient as a maintenance regimen, wherein said titration regimen comprises administering 0.25 mg siponimod at day 1, 0.25 mg at day 2, 0.5 mg at day 3, 0.75 mg at day 4 and 1.25 mg at day 5.
Embodiment 20: A method of ameliorating or preventing a negative chronotropic side effect associated with siponimod treatment of a patient with an autoimmune condition comprising administering an immediate release dosage form comprising 2 mg siponimod once daily to said patient as a maintenance regimen, and
   wherein said patient has experienced a titration regimen beforehand of 0.25 mg siponimod at day 1, 0.25 mg at day 2, 0.5 mg at day 3, 0.75 mg at day 4 and 1.25 mg at day 5.
Embodiment 21: A method of ameliorating or preventing a negative chronotropic side effect associated with siponimod treatment of a patient with an autoimmune condition comprising administering an initial titration regimen of siponimod, and administering an immediate release dosage form comprising 2 mg siponimod once daily to said patient as a maintenance regimen, wherein said titration regimen comprises administering 0.25 mg siponimod at day 1, 0.25 mg at day 2, 0.5 mg at day 3, 0.75 mg at day 4 and 1.25 mg at day 5.
Embodiment 22: A kit containing daily units of medication of siponimod of 0.25 mg, 0.25 mg, 0.5 mg, 0.75 mg and 1.25 mg, wherein the kit contains a dosage form comprising 0.25 mg siponimod.
Embodiment 22a: A kit containing daily units of medication of siponimod of 0.25 mg, 0.25 mg, 0.5 mg, 0.75 mg and 1.25 mg, wherein the kit contains an immediate release dosage form comprising 0.25 mg siponimod, an immediate release dosage form comprising 0.5 mg siponimod and an immediate release dosage form comprising 1 mg siponimod.
Embodiment 23: A kit containing daily units of medication of siponimod of 0.25 mg, 0.25 mg, 0.5 mg, 0.75 mg and 1.25 mg, wherein the kit contains a dosage form comprising 0.25 mg siponimod for use as defined in embodiment 1.
Embodiment 23a: A kit containing daily units of medication of siponimod of 0.25 mg, 0.25 mg, 0.5 mg, 0.75 mg and 1.25 mg, wherein the kit contains an immediate release dosage form comprising 0.25 mg siponimod, an immediate release dosage form comprising 0.5 mg siponimod and an immediate release dosage form comprising 1 mg siponimod for use as defined in embodiment 1.
Embodiment 24: Use of siponimod in the manufacture of a medication for the treatment of a patient with an autoimmune condition wherein an immediate release dosage form comprising 2 mg siponimod is administered once daily to said patient as a maintenance regimen, and wherein said patient has experienced a titration regimen beforehand of 0.25 mg siponimod at day 1, 0.25 mg at day 2, 0.5 mg at day 3, 0.75 mg at day 4 and 1.25 mg at day 5.
Embodiment 25: Siponimod for use in the treatment of an autoimmune disease,
   wherein an immediate release dosage form comprising 2 mg siponimod will be or is administered once daily to a patient as a maintenance regimen, and
   wherein the patient is experiencing or has experienced a titration regimen beforehand of 0.25 mg siponimod at day 1, 0.25 mg at day 2, 0.5 mg at day 3, 0.75 mg at day 4 and 1.25 mg at day 5.
Embodiment 26: Siponimod for use according to embodiment 25, wherein the autoimmune disease is Secondary Progressive Multiple Sclerosis.
Embodiment 27: Siponimod for use in ameliorating or preventing a negative chronotropic side effect associated with siponimod treatment of a patient with an autoimmune condition comprising administering an immediate release dosage form comprising 2 mg siponimod once daily to said patient as a maintenance regimen, and
   wherein said patient has experienced a titration regimen beforehand of 0.25 mg siponimod at day 1, 0.25 mg at day 2, 0.5 mg at day 3, 0.75 mg at day 4 and 1.25 mg at day 5.

Generally, all explanations given above for preferred embodiments of the dosage form of the present invention, e.g. the explanations about preferred EDSS scores, about the preferred composition of the dosage form, about the preferred in-vitro and in-vivo properties of the dosage form, about siponimod salts and particle sizes and the like, also apply to the other aspects of the invention, e.g. the method of treating, the method of ameliorating or preventing a negative chronotropic side effect, the kit and the use in the manufacture of medications.

### Examples

### Example 1: Preparation of immediate release tablets

For the titration/maintenance regimen, 0.25 mg, 0.5 mg, 1 mg and 2 mg siponimod immediate release film-coated tablets can be prepared as described below.

### Process Blending:

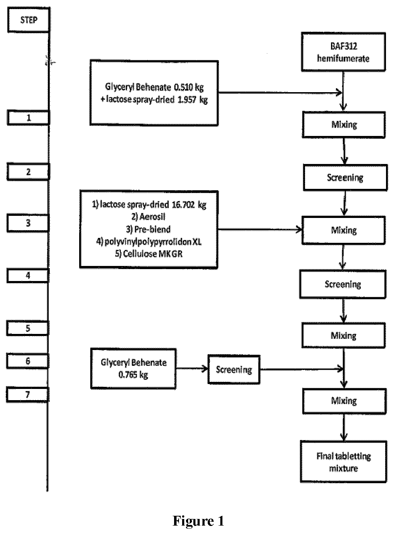

In order to obtain a final mixture ready to be processed to a dosage form, e.g. a tablet, siponimod hemifumarate, e.g. having a X90 value of 18 µm, is blended with different excipients according to the flow diagram of Figure 1. Therefore, siponimod hemifumarate is pre-blended in step 1 with a mixture of glyceryl behenate as moisture protective agent and spray-dried lactose as filler. The pre-blending is carried out in a diffusion mixer Bohle PM400S (L.B. Bohle Maschinen + Verfahren GmbH, Ennigerloh, Germany) for 10 min at 10 rpm. The mixture of step 1 is then sieved in step 2 using a screening mill having a mesh size of 800 µm. The sieved mixture is then blended in step 3 with further spray-dried lactose as filler, Aerosil as glidant, polyvinylpolypyrrolidon XL (crospovidone) as disintegrant and microcrystalline cellulose GR as filler in a diffusion mixer Bohle PM400S for 5 min at 10 rpm. The resulting mixture is again sieved in step 4 using an oscillating screening mill Frewitt GLA ORV having a mesh size of 800 µm and mixed in step 5 in a diffusion mixer Bohle PM400S for 25 min at 10 rpm. In step 6 glyceryl behenate as lubricant, which has been sieved using an oscillating screening mill Frewitt GLA ORV having a mesh size of 800 µm, is added to the mixture of step 5 and mixed in step 7 in a diffusion mixer Bohle PM400S for 10 min at 10 rpm, resulting in the final dosage form mixture.

The final dosage mixture resulted from the blending process is then processed into a dosage form, preferably a tablet. The tablets are formed using a rotary tablet press, a Korsch PH 250 or Korsch XL400 with a compression force of 6 kN. The tablets are then de-dusted with a Krämer deduster (Krämer AG, Switzerland) and finally coated by a perforated pan coater Glatt Coater GC 750 (Glatt GmbH, Germany). Instead of siponimod hemifumarate having a X90 value of 18 µm, siponimod hemifumarate of higher X90 values, e.g. 40-50 µm, or lower X90 values, e.g. 6 µm, can be used.

### Manufacturing Process of Siponimod Film Coated Tablets:

Following the process of Example 1, film-coated tablets with the composition per tablet according to Tables 2 to 5 can be prepared.

**Table 2:**

| Component | Composition per unit [%] | Composition per unit [mg/unit] |
|---|---|---|
| Siponimod hemifumarate* (X90 = 18 µm) | 0.33 | 0.278 |
| Lactose - preblending step 1 | 7.32 | 6.220 |
| Lactose - step 3 | 65.85 | 55.977 |
| Total Lactose | 73.17 | 62.197 |
| Microcryst. cellulose | 15.0 | 12.750 |
| Polyvinylpolypyrrolidon XL | 6.0 | 5.100 |
| Aerosil 200 | 0.50 | 0.425 |
| Glyceryl behenate - step 1 | 2.0 | 1.7 |
| Glyceryl behenate - step 6 | 3.0 | 2.55 |
| Total Glyceryl behenate | 5.0 | 4.250 |
| *Total core tablet* | *100%* | *85.000 mg* |
| Coating premix | 5.134 | 4.6 |
| *Total film coating tablet* | *100%* | *89.600 mg* |

| | | |
|---|---|---|
| * The salt factor is 1.112 | | |

**Table 3:**

| Component | Composition per unit [%] | Composition per unit [mg/unit] |
|---|---|---|
| Siponimod hemifumarate* (X90 = 18 µm) | 0.65 | 0.556 |
| Lactose - preblending step 1 | 7.29 | 6.192 |
| Lactose - step 3 | 65.56 | 55.727 |
| Total Lactose | 72.85 | 61.919 |
| Microcryst. cellulose | 15.0 | 12.750 |
| Polyvinylpolypyrrolidon XL | 6.0 | 5.100 |
| Aerosil 200 | 0.50 | 0.425 |
| Glyceryl behenate - step 1 | 2.0 | 1.7 |
| Glyceryl behenate - step 6 | 3.0 | 2.55 |
| Total Glyceryl behenate | 5.0 | 4.250 |
| *Total core tablet* | *100%* | *85.000 mg* |
| Coating premix | 5.134 | 4.6 |
| *Total film coating tablet* | *100%* | *89.600 mg* |

| | | |
|---|---|---|
| * The salt factor is 1.112 | | |

**Table 4:**

| Component | Composition per unit [%] | Composition per unit [mg/unit] |
|---|---|---|
| Siponimod hemifumarate* (X90 = 18 µm) | 1.31 | 1.112 |
| Lactose - preblending step 1 | 7.22 | 6.136 |
| Lactose - step 3 | 64.97 | 55.227 |
| Total Lactose | 72.19 | 61.363 |
| Microcryst. cellulose | 15.0 | 12.750 |
| Polyvinylpolypyrrolidon XL | 6.0 | 5.100 |
| Aerosil 200 | 0.50 | 0.425 |
| Glyceryl behenate - step 1 | 2.0 | 1.7 |
| Glyceryl behenate - step 6 | 3.0 | 2.55 |
| Total Glyceryl behenate | 5.0 | 4.250 |
| *Total core tablet* | *100%* | *85.000 mg* |
| Coating premix | 5.134 | 4.6 |
| *Total film coating tablet* | *100%* | *89.600 mg* |

| | | |
|---|---|---|
| * The salt factor is 1.112 | | |

**Table 5:**

| Component | Composition per unit [%] | Composition per unit [mg/unit] |
|---|---|---|
| Siponimod hemifumarate* (X90 = 18 µm) | 2.62 | 2.224 |
| Lactose - preblending step 1 | 7.09 | 6.025 |
| Lactose - step 3 | 63.79 | 54.226 |
| Total Lactose | 70.88 | 60.251 |
| Microcryst. cellulose | 15.0 | 12.750 |
| Polyvinylpolypyrrolidon XL | 6.0 | 5.100 |
| Aerosil 200 | 0.50 | 0.425 |
| Glyceryl behenate - step 1 | 2.0 | 1.7 |
| Glyceryl behenate - step 6 | 3.0 | 2.55 |
| Total Glyceryl behenate | 5.0 | 4.250 |
| *Total core tablet* | *100%* | *85.000 mg* |
| Coating premix | 5.134 | 4.6 |
| *Total film coating tablet* | *100%* | *89.600 mg* |

| | | |
|---|---|---|
| * The salt factor is 1.112 | | |

### Example 2: In-vitro release of immediate release tablets

For the dissolution tests, a USP dissolution apparatus 2 (paddle) has been used.

The dissolution conditions are summarized in Table 6 below. The dissolution tests has been carried out according to USP <711> "Dissolution".

**Table 6:**

| | |
|---|---|
| Speed of rotation | 60 ± 2 rpm |
| Test medium | Phosphate buffer pH 6.8 + 0.1% (m/v) Tween 80 |
| Volume of test medium | 500 mL for the 0.25 mg dosage strength |
| | 900 mL for the 0.5, 1 and 2 mg dosage strengths |
| Temperature | 37 ± 0.5°C |

The dissolution rates of the siponimod tablets of Example 1 are summarized in Table 7 below.

**Table 7:**

| **Dosage strength** | **Dissolution rate [%] after** | | | | | |
|---|---|---|---|---|---|---|
| | **5min** | **15min** | **30min** | **45min** | **60min** | **75min** |
| 0.25 mg | 34% | 92% | 99% | 100% | 100% | 100% |
| 0.5 mg | 36% | 91% | 98% | 99% | 99% | 99% |
| 1 mg | 37% | 87% | 97% | 99% | 100% | 100% |
| 2 mg | 50% | 87% | 96% | 98% | 99% | 100% |

According to the results in Table 7, the in-vitro release of siponimod is an immediate release.

### Example 3: Data from animal models

### Example 3.1: Level of siponimod in cerebrospinal fluid (CSF) in mice

Female C57B1/6 mice were treated daily with 3 mg/kg BAF312, p.o. for 8 days. 8 hours after the last administration, animals were sacrificed and the levels of BAF312 were measured in blood, brain and CSF.

Data is summarized in Table 8 below. Shown are mean values of 3-5 animals and standard error of the mean (in brackets).

**Table 8:**

| Strain and species | | | | conc BAF312 (nM) (SEM) | | |
|---|---|---|---|---|---|---|
| | Dose (mg/kg, qd) | day | sampling time | Blood | Brain | CSF |
| C57B1/6 mice | 3 | 8 | 8h | 973.3 (62.1) | 5552 (298) | 7.2(1) |
| C57B1/6 mice, EAE | 3 | 8 | 8h | 1600 (79) | 9193 (310) | 17.7 (3.2) |

This shows that a clinically relevant dose of BAF312 (3 mg/kg in mice) leads to exposures in the CSF above the EC₅₀ for S1P1 (0.4 nM) and S1P5 (1 nM).

### Example 3.2: Use of siponimod to treat relapse and progression in PLP₁₃₉₋₁₅₁ induced experimental autoimmune encephalomyelitis (EAE) in SJL/J mice

Female SJL/J mice were immunized with 50 µg PLP₁₃₉₋₁₅₁ in CFA, followed by one injection of 200 ng Pertussis toxin, i.p.. Three days after the first clinical symptoms, the mice were randomized to either vehicle or BAF312 and treated daily with 3 mg/kg BAF312 p.o..

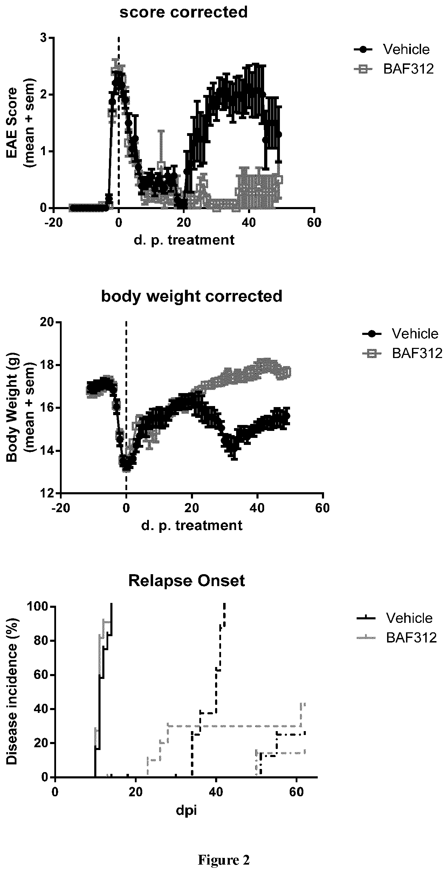

Results are shown in Figure 2. Treatment with BAF312 strongly inhibited the occurrence of subsequent progression (EAE score- and body weight-diagrams) and relapses (relapse onset-diagram). In this specific EAE model, the mice normally recover well from the initial phase of disease, but fail to recover fully from any subsequent disease phase.

### Example 3.3: PET imaging in primate brain

An [¹²³I]-labelled analogue of siponimod, [¹²³I]-compound A (radioactive half-life 13.2 hours), was used to investigate siponimod distribution in non-human primates (NHP). Despite structural modification to introduce the radiolabel, all affinity, selectivity and rat pharmacokinetic properties of compound A were shown to be similar to those of BAF312 (see Example 3.3.2).

### Example 3.3.1: Synthesis of PET ligand

### General:

All chemicals, reagents and solvents for the synthesis of the compounds were analytical grade, purchased from commercial sources and used without purification, unless otherwise specified.

¹H NMR spectra were acquired on a Bruker (400 MHz), or Bruker Advance (600 MHz). δ values are given in parts per million (ppm) relative to the residual solvent peak. Coupling constants (*J*) are given in Hz, spectra splitting pattern are designated as singulet (*s*), doublet (*d*), doublet doublet (*dd*)*,* triplet (*t*)*,* quadruplet (*q*)*,* multiplet or more overlapping signals (*m*)*,* broad signal (*br*). Solvents are given in parentheses.

### Analytical LCMS/HPLC conditions (% = percent by volume)

UPLC-ZQ2000, column Acquity HSS-T3 1.8 µm; 2.1 x 50 mm; gradient: A, water + 5 % acetonitrile + 0.5-1.0% HCO₂H; B, acetonitrile + 0.5-1.0% HCO₂H; from 98/2 to 2/98 in 4.3 min + 0.7 min isocratic; flow rate 1.0 ml/min, Rt = retention time

### Preparative HPLC:

Gilson Trilution LC, Column: SunFire C18, 30 x 100mm, 5 µm, Eluent: Water (+0.1% TFA): acetonitrile (+ 0.1% TFA) from 85:15 to 65:35 in 16 min; flow 50 mL/min.

### Synthesis of intermediates:

**Step 1: Methyl 4-cyclohexyl-3-iodobenzoate is** prepared according to the procedure described by Zhijian Liu, J. Org. Chem. 2007, 72, 223-232 and Laurence Burgess, Synthetic Communications 1997, 27, 2181-2191. 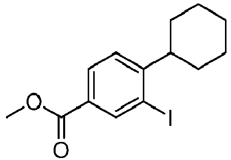 ¹H NMR (400 MHz, CDCl₃): δ = 8.40 (d, 1H, *J* = 1.5 Hz), 7.89 (dd, 1H, *J* = 1.5, 8.1 Hz), 7.18 (d, *J* = 8.1 Hz), 3.80 (s, 3H), 2.75 (tt, 1H, J = 11.8, 3.4 Hz), 1.86-1.66 (m, 5H), 1.48-1.34 (m, 5H); ¹³C NMR (400 MHz, CDCl₃): δ = 167.3, 143.4, 140.5, 129.6, 128.2, 126.5, 98.1, 52.7, 48.8, 34.0, 27.0, 26.5; LC-MS: t = 1.55 min, mass not detected

### Step 2: (4-cyclohexyl-3-iodophenyl)methanol

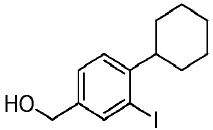

LiBH₄ (2M / THF) (0.753 ml, 1.506 mmol) is added at 0°C to a solution of methyl 4-cyclohexyl-3-iodobenzoate (610 mg, 1.506 mmol) in THF (20 mL) and the mixture is stirred at RT for 18 hours. Then two consecutive addition of LiBH₄ (2M / THF) (0.753 ml, 1.506 mmol) are performed until completion of the reaction. The reaction mixture is quenched with a saturated Na₂SO₄ solution, stirred vigorously at RT for 1h, filtered over celite and concentrated. The crude product is purified by flash chromatography using cHex/EtOAc (from 100:0 to 70:30) as eluent to give the title compound as a colorless oil (81% yield, 85% pure). (4-cyclohexyl-3-iodophenyl)methanol is used as such in the next step. LC-MS: t = 1.29 min, mass not detected

### Step 3: 4-cyclohexyl-3-iodobenzyl methanesulfonate

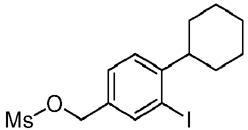

Triethylamine (0.222 mL, 1.594 mmol) and methanesulfonyl chloride (0.114 ml, 1.461 mmol) are added to a solution of (4-cyclohexyl-3-iodophenyl)methanol (420 mg, 1.328 mmol) in CH₂Cl₂ (15 mL) under argon at 0°C and the resulting mixture is stirred at 0°C for 1 hour. The product is extracted with H₂O/ CH₂Cl₂, the organic layers are dried over Na₂SO₄, filtered and concentrated. The crude product is purified by flash chromatography using cHex/EtOAc (from 100:0 to 70:30) as eluent to afford the title compound as a colorless oil (465 mg, 78%).
¹H NMR (400 MHz, CDCl₃): δ = 7.89 (d, 1H, J = 1.52 Hz), 7.38 (dd, 1H, J = 1.5, 7.9 Hz), 7.25 (d, 8 Hz), 5.16 (s, 2H), 2.99 (s, 3H), 2.81 (tt, 1H, J = 2.8, 8.8 Hz), 1.94-1.76 (m, 5H), 1.54-1.22 (m, 5H); ¹³C NMR (400 MHz, CDCl₃): δ = 144.06, 139.40, 135.60, 129.1, 127.0, 99.31, 70.4, 38.7, 34.0, 33.8, 26.6, 25.9; LC-MS: t = 1.36 min, m/z 412.1 ([M+H₂O])

### Step 4: (E)-ethyl N-(4-cyclohexyl-3-iodobenzyl)oxyacetimidate

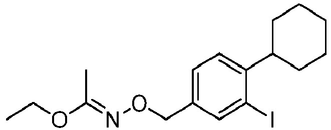

Sodium hydride (70.4 mg, 1.613 mmol) is added to a solution of (Z)-ethyl-N-hydroxyacetamidate (166 mg, 1.613 mmol) in DMF (5 mL) at RT under argon and the mixture is stirred at RT for 20 min. Then a solution of 4-cyclohexyl-3-iodobenzyl methanesulfonate (530 mg, 1.344 mmol) in DMF (1mL) is added and the resulting mixture is stirred at RT for 1 hour. The mixture is quenched at 0°C with NH₄Cl, extracted with EtOAc and the organic layers are dried over Na₂SO₄, filtered and concentrated. The crude is purified by flash chromatography on silica gel using cHex/EtOAc (from 100:0 to 96:4) as eluent to afford the title compound as a colorless oil (490 mg, 89%)
¹H NMR (400 MHz, CDCl₃): δ = 7.86 (s, 1H), 7.32 (d, 1H, J = 7.3 Hz), 7.19 (d, 1H, J = 7.9 Hz), 4.84 (s, 2H), 4.02 (q, 2H, J = 7.04 Hz), 2.79 (tt, 1H, J = 2.9, 11.7 Hz), 1.96 (s, 3H), 1.94-1.76 (m, 5H), 1.56-1.25 (m, 5H); ¹³C NMR (400 MHz, CDCl₃): δ = 162.7, 143.6, 139.4, 134.1, 128.3, 126.5, 98.8, 62.6, 48.8, 33.4, 26.7, 26.3, 14.5, 14.0; LC-MS: t = 1.67 min, m/z 402.2 ([M+H])

### Synthesis of non-labelled PET-ligand (compound A):

(E)-1-(4-(1-(((4-cyclohexyl-3-iodobenzyl)oxy)imino)ethyl)-2-ethylbenzyl)azetidine-3 -carboxylic acid (compound A) is prepared according to the experimental section described by Pan et al. (ACS Med Chem Lett, 2013, 4, 333-337).

### Step 1: (E)-1-(3-ethyl-4-(hydroxymethyl)phenyl)ethanone O-(4-cyclohexyl-3-iodobenzyl)oxime

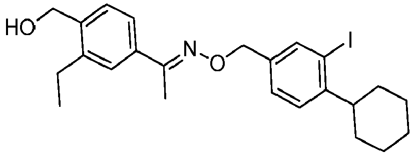

¹H NMR (400 MHz, CDCl₃): δ = 7.93 (s, 1H), 7.46-7.55 (m, 2H), 7.34-7.43 (m, 2H), 7.21 (d, J=7.9 Hz, 1H), 5.16 (s, 2H), 4.76 (d, J=5.6 Hz, 2H), 2.71-2.84 (m, 3H), 2.28 (s, 3H), 1.75-1.95 (m, 4H), 1.60-1.65 (m, 1H), 1.32-1.50 (m, 5H), 1.22 (s, J=7.9 Hz, 3H); ¹³C NMR (400 MHz, CDCl₃): δ = 160.5, 141.7, 141.2, 143.5, 139.5, 134.5, 133.5, 127.9, 126.9, 126.2, 123.5, 92.8, 74.7, 62.3, 48.5, 33.4, 27.8, 25.9, 26.3, 15.3, 13.1; LC-MS: t = 1.62 min, m/z 492.5 ([M+H])

### Step 2: (E)-1-(4-(1-(((4-cyclohexyl-3-iodobenzyl)oxy)imino)ethyl)-2-ethylbenzyl)azetidine-3-carboxylic acid (compound A)

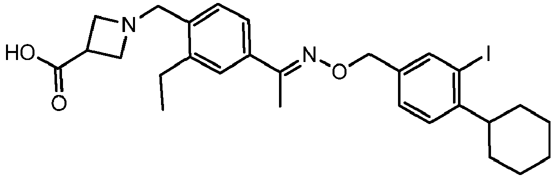

¹H NMR (600 MHz, DMSO): δ = 7.87 (s, 1 H) 7.45 (s, 1H) 7.38-7.42 (m, 2H) 7.23-7.29 (m, 2H) 5.10 (s, 2H) 3.57 (s, 2H), 3.39-3.43 (m, 2H) 3.20-3.24 (m, 3H) 2.63-2.71 (m, 3H) 2.19 (s, 3H) 1.73-1.82 (m, 4H) 1.67-1.73 (m, 1H) 1.31-1.37 (m, 4H) 1.21-1.26 (m, 1H) 1.16 (t, J=7.5 Hz, 3H); ¹³C NMR (600 MHz, DMSO): δ = 174.3, 154.7, 148.0, 142.3, 138.7, 138.0, 136.6, 134.6, 128.6, 128.5, 126.5, 125.7, 123.3, 101.2, 73.9, 59.5, 56.6, 47.8, 33.6, 32.9, 26.4, 25.5, 24.8, 15.1, 12.6; ; LC-MS: t = 1.27 min, m/z 575.3 ([M+H])

### PET-ligand precursor synthesis:

### Step 1: 4-{(1E)-N-[(4-Cyclohexyl-3-iodophenyl)methoxy]ethanimidoyl}-2-ethylbenzaldehyde

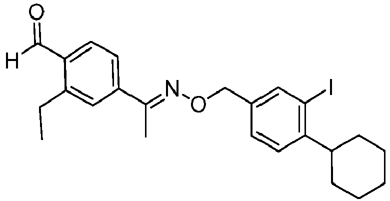

A 50 ml of three-necked flask is charged with (4-{(1E)-N-[(4-Cyclohexyl-3-iodophenyl)methoxy]ethanimidoyl}-2-ethylphenyl)methanol (22.95 g, 89% wt., 41.57 mmol) and MnO₂ (36.13 g, 415.66 mmol) at RT, followed by 1,4-dioxane (230 mL) to give a black suspension. The resulting mixture is heat up to 60 °C for 1 hour then at 100 °C for 1.5 hour and cooled to RT. The mixture is filtered through a pad of microcrystalline cellulose and the filtration cake is washed with isopropyl acetate (230 mL) twice. The combined filtrates are removed under reduced pressure to obtain the title compound (20.2 g, yield 99%) as yellow solid, which could be used in next step without further purification.

### Step 2: 4-[(1E)-N-{[4-Cydohexyt-3-(4,4,5,5-tetramethyl-1,3,2-dioxaborotan-2-yl)phenyl]methoxy}ethanimidoyl]-2-ethylbenzaldehyde

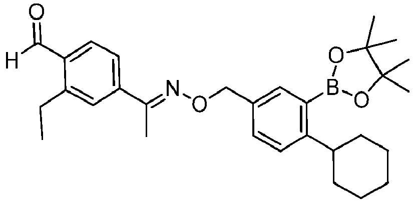

A 100 ml of three-necked flask is charged with 4-{(1E)-N-[(4-Cyclohexyl-3-iodophenyl)methoxy]ethanimidoyl}-2-ethylbenzaldehyde (6.0 g, 12.26 mmol) and bis(pinacolato)diboron (4.67g, 18.39 mmol) at RT. Then dimethylacetamide (60 mL), KOAc (3.61 g, 36.78 mmol) and PdCl₂(dppf) (0.897 g, 1.23 mmol) are added sequentially in this order. The whole system is purged with nitrogen three times and the reaction mixture is heated at 100 °C under nitrogen for 3 hours, then cooled to RT. Isopropyl acetate (180 mL) is added and the mixture is filtered through a pad of microcrystalline cellulose. The filtration cake is washed with isopropyl acetate (60 mL). The filtrate is washed with 1 N HCl (120 mL) and aqueous layers are re-extracted with isopropyl acetate (60 mL). The combined organic layers are washed with 10% NaCl aq. (120 mL) three times. Organic solvents are concentrated under reduced pressure to afford crude title compound (9.0 g) as black oil, which is purified through silica gel column chromatography (eluent: Heptane → Heptane/EtOAc = 50:1 → Heptane/EtOAc = 25:1, v/v). The pure product (4.8 g) is obtained as a white solid (80% yield).
¹H NMR (400 MHz, CDCl₃): δ = 10.28 (s, 1H), 7.81 (d, J = 8 Hz, 1H), 7.77 (s, 1H), 7.60 - 7.63 (m, 1H), 7.58 (s, 1H), 7.43 -7.46 (m, 1H), 7.25 - 7.31 (m, 1H), 5.23 (s, 2H), 3.2 - 3.35 (m, 1H), 3.08 (q, J = 8 Hz, 2H), 2.25 (s, 3H), 1.6 - 1.8 (m, 4H), 1.4 - 1.55 (m, 1H), 1.3 - 1.45 (m, 5H), 1.36 (s, 12H), 1.29 (t, J = 8 Hz, 3H); MS: m/z 490.31 ([M+H])

### Step 3: 1-({4-[(1E)-N-{[4-Cyclohexyl-3-(4,4,5,5-tetramethyl-1,3,2-dioxaborolan-2-yl)phenyl]methoxy}ethanimidoyl]-2-ethylphenyl}methyl)azetidine-3-carboxylic acid

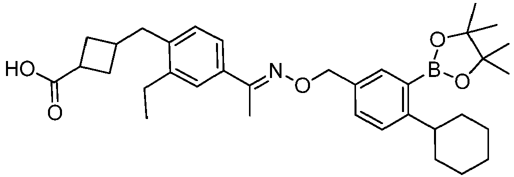

A 50 mL of three-necked flask is charged with azetidine-3-carboxylic acid (1.11 g, 1.0 mmol) and acetic acid (7.54 mL) at RT to give a yellow solution, followed with CH₂Cl₂ (16.8 mL). 4-[(1E)-N-{[4-cyclohexyl-3-(4,4,5,5-tetramethyl-1,3,2-dioxaborolan-2-yl)phenyl]methoxy}ethanimidoyl]-2-ethylbenzaldehyde (2.8 g, 96% wt, 5.49 mmol) solution in CH₂Cl₂ (28 mL) is added in one portion and the mixture is stirred at RT for 30 minutes. Then the mixture is cooled to 3°C and triethylamine (21.4 mL) is added in 30 minutes. This addition is exothermic and the temperature is kept below 10°C. The stirring is pursued for 2 hours at RT. NaBH(OAc)₃ (2.33g, 11.0 mmol) is added in one portion and the yellow suspension stirred at RT for 16 hours. The mixture is cooled down to 0°C and quenched with water (22 mL) (exothermic). The organic layer is separated and water layers are extracted with CH₂Cl₂ (17 mL). Combined organic layers are washed with 1.5 N HCl (35 mL) and 95% ethanol (18 mL) is added to resolve the resulting emulsion. The organic layer is separated, further washed with NH₄C1 aq. (including 5 mL sat. NH₄Cl + 2 mL H₂O), 0.5 N NaOH (16.8 mL) and water (17 mL). The organic solvents are removed under reduced pressure to afford the title compound (2.5 g) as yellow solid with yield 77%.
¹H NMR (400 MHz, CDCl₃): δ = 7.75 (s, 1H), 7.53 (s, 1H), 7.45 - 7.48 (m, 2H), 7.35 - 7.46 (m, 1H), 7.25 -7.30 (m, 1H), 5.19 (s, 2H), 4.16 (s, 2H), 4.05 - 4.15 (m, 2H), 3.9 - 4.05 (m, 2H), 3.3 - 3.4 (m, 1H), 3.2 - 3.3 (m, 1H), 2.73 (q, J = 8 Hz, 2H), 2.22 (s, 3H), 1.8 - 1.9 (m, 4H), 1.7 - 1.8 (m, 1H), 1.37 - 1.45 (m, 5H), 1.35 (s, 12H), 1.21 (t, J = 8 Hz, 3H); MS: m/z 575.37 ([M+H])

### Step 4: (E)-(5-((((1-(4-((3-carboxyazetidin-1-yl)methyl)-3-ethylphenyl)ethylidene)amino)oxy)methyl)-2-cyclohexylphenyl)trifluoroborate

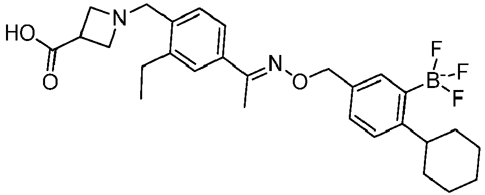

A 50 ml of three-necked flask is charged with 1-({4-[(1E)-N-{[4-Cyclohexyl-3-(4,4,5,5-tetramethyl-1,3,2-dioxaborolan-2-yl)phenyl]methoxylethaniniidoyl]-2-ethylphenyllmethyl)azetidine-3-carboxylic acid (0.49 g, 0.853 mmol) and MeOH (7.5 mL) at RT. KHF₂ aq. (4.5 M, 1.1 mL, 4.78 mmol) is added in one portion. The reaction mixture is stirred at RT for 15 min. The reaction mixture is cooled to 0 - 5°C. Water (21 mL) is added dropwise and the reaction mixture is stirred at ice-water bath for 1.5 hours. The suspension is filtered and the filtration cake is rinsed with water (2 mL) and CH₃CN (2 mL). The cake is dried under vacuum at RT for 16 hours to afford the title compound (0.43 g, yield 88%) as white solid.
¹H NMR (600 MHz, DMSO): δ = 7.48 (s, 1H), 7.37-7.46 (m, 2H), 7.28 (d, *J*=7.7 Hz, 1H), 7.01-7.15 (m, 2H), 5.05 (s, 2H), 3.60 (s, 2H), 3.34-3.52 (m, 2H), 3.13-3.29 (m, 4H), 2.67 (d, *J*=7.7 Hz, 2H), 2.61-2.78 (m, 1H), 2.17 (s, 3H), 1.67-1.77 (m, 5H), 1.16 - 1.34 (m, 5H), 1.18 (s, 3H); ¹³C NMR (600 MHz, DMSO): δ = 174.25, 153.57, 151.04, 142.26, 136.27, 135.01, 132.70, 131.82, 128.66, 125.74 (2C), 123.91, 123.27, 76.84, 59.46, 56.58 (2C), 40.93, 34.60 (2C), 33.62, 26.97 (2C), 26.19, 24.83, 15.12, 12.57; ¹⁹F NMR (600 MHz, DMSO): δ = -135.36 (s, BF₃)

### General procedure for the preparation of PET-ligand labeled with ¹²³I:

Na¹²³I (2110 MBq), water for injection, glacial acetic acid, trifluoroborate precursor and chloramine-T are placed into a 2 mL Wheaton vial and allowed to react for 10 min at RT. The reaction is quenched with a solution of sodium metabisulfite in saturated sodium bicarbonate. The resulting mixture is diluted with the mobile phase and purified by HPLC to provide after formulation 1184 MBq of labeled product.

### Example 3.3.2: In-vitro affinity of compound A compared to BAF312

Despite structural modification to introduce the radiolabel, all affinity, selectivity and rat pharmacokinetic properties of compound A were shown to be similar to those of BAF312. BAF312 had an EC₅₀ for cloned human S1P1 and S1P5 of 0.90 nM and 0.79 nM, respectively. Compound A had an EC₅₀ for cloned human S1P1 and S1P5 of 2.20 nM and 1.35 nM, respectively.

### Example 3.3.3: PET data from primate brain

### Methods:

[¹²³I]-compound A was prepared from its potassium trifluoroborate precursor MS992, by reaction with [¹²³I]NaI in presence of chloramine-T and acetic acid at room temperature for 20 min. After HPLC purification and formulation the radiochemical yield was 57 ± 16 % (n= 4) with a radiochemical purity greater than 95%.

[¹²³I]-compound A was administered to 2 adult male rhesus NHPs (*Macaca mullata*)*,* as single intravenous bolus. Single photon emission computed tomography (SPECT) studies were performed using a MollyQ camera (Neurophysics Inc., Shirley, MA, USA). Brain penetration was assessed by serial dynamic scanning over a 2 day period following radiotracer injection. Scan duration was approximately 8 hours on day 1 and 2 hours on day 2 with a 24 hour interval between the last scan on day 1 and the first scan on day 2. SPECT images were corrected for motion, decay and tissue attenuation. The scans were reconstructed and then analyzed using PMOD 3.203 software. Standardized uptake values (SUV) were calculated by normalizing for injected activity and body weight.. Then, they were co-registered to a magnetic resonance imaging template for volumes of interest extraction, time-activity curves generation and brain penetration estimation. Blood samples were taken to determine the radio-metabolite concentration in plasma

### Results:

Following intravenous bolus injection, [¹²³I]-compound A penetrated NHP brain with highest concentration in brain of 0.008-0.014 %ID/mL at around 24 hours post-injection. Peak SUV values of around 1.4-1.8 were also determined during day 2 imaging session. Radiotracer metabolism in plasma was slow and at 24 hours post-injection ∼70% of parent compound was still present in rhesus monkey plasma. This demonstrates that the structurally closely related siponimod analogue is able to penetrate into the primate brain and reaches significant drug levels in the brain.

### Example 4: Clinical study

A multicentre, randomized, double-blind, parallel-group, placebo-controlled variable treatment duration study evaluating the efficacy and safety of siponimod in patients with Secondary Progressive Multiple Sclerosis.

### 1. Study objectives

### a) Primary objective

The primary objective is to demonstrate the efficacy of siponimod relative to placebo in delaying the time to 3-month confirmed disability progression in patients with SPMS as measured by EDSS.

### b) Key secondary objectives

The first key secondary objective is to demonstrate the efficacy of siponimod relative to placebo in delaying the time to 3-month confirmed worsening of at least 20% from the baseline in the timed 25-foot walk test (T25-FW).

The second key secondary objective is to demonstrate the efficacy of siponimod relative to placebo in reducing the increase in T2 lesion volume from the baseline to the end of the study.

### c) Further secondary objectives include

- To evaluate the efficacy of siponimod relative to placebo in delaying the time to 6-month confirmed disability progression as measured by EDSS
- To evaluate the efficacy of siponimod relative to placebo in reducing the frequency of confirmed relapses as evaluated by the annualized relapse rate (ARR), and to evaluate time to first relapse and proportion of relapse-free patients
- To evaluate the effect of siponimod compared to placebo on the patient reported outcome Multiple Sclerosis Walking Scale (MSWS-12)
- To evaluate the efficacy of siponimod compared to placebo with respect to inflammatory disease activity and burden of disease, as measured by conventional MRI (T1 Gd-enhancing lesions, new or enlarging T2 lesions, brain volume)
- To evaluate the safety and tolerability of siponimod vs. placebo

### d) Exploratory objectives include

- To evaluate the effect of siponimod compared to placebo on the following patient-reported outcomes:
   - The health-related quality of life (QoL) as measured by the Multiple Sclerosis Impact Scale (MSIS-29)
   - The health-related quality of life (QoL) as measured by the EQ-5D
- To explore efficacy of siponimod relative to placebo on defined cognitive tests:
   - Paced Auditory Serial Addition Test (PASAT)
   - Symbol Digit Modalities Test (SDMT)
   - Brief Visuospatial Memory Test Revised (BVMTR)
- To evaluate the efficacy of siponimod relative to placebo in the evolution of acute lesions into chronic black holes by MRI
- To evaluate the efficacy of siponimod relative to placebo on the MSFC z-score
- To evaluate the efficacy of siponimod relative to placebo in delaying the time to:
   - 3-month confirmed worsening of at least 20% from the baseline in the timed 25-foot walk test (T25W) or
   - 3-month confirmed disability progression as measured by EDSS score or
   - 3-month confirmed worsening of at least 20% from the baseline in the 9-hole peg test (9-HPT) in either one of the hands (dominant or non-dominant).
- To explore the relationship between disability progression endpoints and drug concentration/lymphocyte count
- To explore the relationship between selected safety parameters and drug concentration/lymphocyte count
- To evaluate the pharmacokinetics of siponimod

### 2. Population

The study population consists of ambulatory patients with an EDSS score of 3.0 to 6.5 and aged between 18 to 60 years with a diagnosis of MS with a secondary progressive disease course (SPMS).

### 2.1. Inclusion Criteria

Patients eligible for inclusion in this study have to fulfill **all** of the following criteria:
1. Written informed consent must be obtained before any assessment is performed.
2. Male or female patients aged 18 to 60 years (inclusive)
3. Prior history of relapsing-remitting MS (RRMS) according to the 2010 Revised McDonald criteria (Polman et al. 2011)
4. Secondary progressive course of MS (SPMS), defined by a progressive increase in disability (of at least 6 months duration) in the absence of relapses or independent of, relapses (Lublin et al. 1996, 2003, Rovaris et al. 2006)
   - Attestation by the investigator in a written statement or that the disease has entered the progressive stage (according to the study definition) at least 6 months prior to enrollment
5. Disability status at Screening with an EDSS score of 3.0 to 6.5(inclusive)
6. Documented EDSS progression in the 2 years prior to study of ≥1 point for patients with EDSS <6.0 at baseline, and ≥0.5 point for patients with EDSS ≥6.0 at baseline. Should documented EDSS scores not be available, a written summary of the clinical evidence of disability progression in the previous 2 years, and retrospective assessment of EDSS score from data up to 2 years prior to screening must be submitted for central review.
7. No evidence of relapse or corticosteroid treatment within 3 months prior to randomization

### 2.2. Exclusion Criteria

Patients fulfilling **any** of the following criteria are not eligible for inclusion in this study.
1. Patients with an active chronic disease (or stable but treated with immune therapy) of the immune system other than MS (e.g. rheumatoid arthritis, scleroderma, Sjogren's syndrome, Crohn's disease, ulcerative colitis, etc.) or with a known immunodeficiency syndrome (AIDS, hereditary immune deficiency, drug-induced immune deficiency).
2. Pregnant or nursing (lactating) women, where pregnancy is defined as the state of a female after conception and until the termination of gestation, confirmed by a positive hCG laboratory test.
3. Women of child-bearing potential, defined as all women physiologically capable of becoming pregnant, unless they are using highly effective methods of contraception during dosing and for 7 days after the last dose of BAF312. Highly effective contraception methods include:
   - Total abstinence (when this is in line with the preferred and usual lifestyle of the subject. Periodic abstinence (e.g., calendar, ovulation, symptothermal, post-ovulation methods) and withdrawal are not acceptable methods of contraception
   - Female sterilization (have had surgical bilateral oophorectomy with or without hysterectomy) or tubal ligation at least six weeks before taking study treatment. In case of oophorectomy alone, only when the reproductive status of the woman has been confirmed by follow-up hormone level assessment is she considered not of child bearing potential.
   - Male sterilization (at least 6 months prior to screening). For female subjects on the study, the vasectomized male partner should be the sole partner for that subject
   - Combination of any two of the following:
      a. Use of oral, injected or implanted hormonal methods of contraception or other forms of hormonal contraception that have comparable efficacy (failure rate <1%), for example hormone vaginal ring or transdermal hormone contraception.
      b. Placement of an intrauterine device (IUD) or intrauterine system (IUS)
      c. Barrier methods of contraception: Condom or Occlusive cap (diaphragm or cervical/vault caps) with spermicidal foam/gel/film/cream/vaginal suppository

In case of use of oral contraception women should have been stable on the same pill for a minimum of 3 months before taking study treatment.

Women are considered post-menopausal and not of child bearing potential if they have had 12 months of natural (spontaneous) amenorrhea with an appropriate clinical profile (e.g. age appropriate, history of vasomotor symptoms) or have had surgical bilateral oophorectomy (with or without hysterectomy) or tubal ligation at least six weeks prior to screening. In the case of oophorectomy alone, only when the reproductive status of the woman has been confirmed by follow up hormone level assessment is she considered not of child bearing potential.
4. History of malignancy of any organ system (other than localized basal cell carcinoma of the skin), treated or untreated, within the past 5 years, regardless of whether there is evidence of local recurrence or metastases.
5. Diabetes mellitus, unless well controlled and without known organ complications such as reduced renal function, significant retinal pathology or neuropathy.
6. Diagnosis of macular edema during pre-randomization phase (patients with a history of macular edema will be allowed to enter the study provided that they do not have macular edema at the ophthalmic examination at the Screening Visit)
7. Patients with active systemic bacterial, viral or fungal infections, or known to have AIDS or to have positive HIV antibody.
8. Positive results of screening period testing for serological markers for hepatitis A, B, C, and E indicating acute or chronic infection:
   - anti-HAV IgM
   - HBs Ag and/or anti-HBc IgM
   - anti-HCV IgG or IgM
   - anti-HEV IgM (if positive IgG, perform HEV-RNA PCR and if negative, patient can be enrolled in study).
9. Negative for varicella-zoster virus IgG antibodies at Screening
10. Have received any live or live-attenuated vaccines (including for varicella-zoster virus or measles) within 2 months prior to randomization
11. Have been treated with any of the medications listed below:
   - BAF312 at any time
   - fingolimod within 2 months prior to randomization, or have received treatment on fingolimod for more than 6 months
   - intravenous immunoglobulin within 2 months prior to randomization
   - natalizumab within 6 months prior to randomization
   - immunosuppressive/chemotherapeutic medications (e.g. azathioprine, methotrexate) within 6 months prior to randomization
   - cyclophosphamide within 1 year prior to randomization
   - rituximab, ofatumumab, ocrelizumab, cladribine within 2 years prior to randomization
   - alemtuzumab at any time
   - any mitoxantrone during previous 2 years prior to randomization or evidence of cardiotoxicity following mitoxantrone or a cumulative life-time dose of more than 60 mg/m²
   - lymphoid irradiation, bone marrow transplantation or other immunosuppressive treatments with effects potentially lasting over 6 months, at any time
12. Patients with any medically unstable condition as determined by the investigator.
13. Any of the following cardiovascular conditions:
   - History of or current significant cardiac disease including cardiac failure (NYHA functional class II-IV), myocarditis, cardiomyopathy, angina pectoris or myocardial infarction (within 6 months), unstable angina (within 6 months), stroke (within 6 months), TIA (within 6 months), decompensated heart failure requiring hospitalization (within 6 months) or uncontrolled arterial hypertension.
   - Cardiac conduction or rhythm disorders including complete left bundle branch block, sinus arrest or sino-atrial block, symptomatic bradycardia, sick sinus syndrome, Mobitz Type II second degree AV-block or higher grade AV-block (either history or observed at screening), unless patient has a functioning pacemaker
   - Cardiac arrhythmias requiring treatment or a history of cardiac syncope.
   - Patients receiving treatment with Class Ia or III antiarrhythmic drugs (e.g., quinidine, disopyramide, amiodarone, bretylium, sotalol, ibulitide, azimilide, dofelitide, ajmaline, procainamide).
   - Conditions requiring treatment with medication that may cause AV block and suppress AV conduction (e.g. beta-blockers, carbamazepine, lamotrigine, non-dihydropyridine calcium-channel blockers, or cardiac glycosides)
   - Patients receiving at randomization (at treatment initiation) beta blockers, heart-rate slowing calcium channel blockers (ivadrabine, verapamil or diltiazem), or other substances which may decrease heart rate such as digoxin, anticholinesteratic agents or pilocarpine.
   - PR interval >230 msec. Long QT syndrome or QTcF prolongation >450 msec in males or >470 msec in females, on screening electrocardiogram (ECG)
   - Severe autonomic nervous system dysfunction
   - Cardiac condition requiring catheter ablation
   - Other conditions or treatments that may significantly impact the safety of the patient as determined by the investigator
14. Any of the following pulmonary conditions:
   - History of or active severe respiratory disease, including COPD or pulmonary fibrosis
   - tuberculosis, except for history of successfully treated tuberculosis or history of prophylactic treatment after positive PPD skin reaction
   - patients with severe asthma or asthma requiring regular treatment with oral steroids
15. Patients with any of the following hepatic conditions prior to randomization:
   - History of alcohol abuse, chronic liver or biliary disease
   - Total or conjugated bilirubin greater than 1.5 times ULN range, unless in the context of Gilbert's syndrome
   - Alkaline phosphatase (AP) greater than 1.5 times the ULN range
   - AST (SGOT), ALT (SGPT) or Gamma-glutamyl-transferase (GGT) greater than 3 times the ULN range
16. Any of the following abnormal laboratory values prior to randomization:
   - serum creatinine > 1.7 mg/dL (150 µmol/L)
   - white blood cell (WBC) count < 3,500/mm³ (< 3.5 x 10⁹/L)
   - lymphocyte count < 800/mm³ (< 0.8 x 10⁹/L)
   - serum potassium > ULN
   - or other clinically significant laboratory assessment (i.e. hypomagnesemia or hypokalemia)
17. Patients with any of the following neurologic/psychiatric disorders prior to randomization:
   - C-SSRS Suicidal ideation score of 4 or above, or any response of "yes" on the suicidal behavior item that is related to suicide behavior occurring during the last 2 years (if this criteria is met, the patient must immediately be referred to the health care professional for further assessment and/or treatment);
   - history of substance abuse (drug or alcohol) or any other factor (i.e., serious psychiatric condition) that may interfere with the subject's ability to cooperate and comply with the study procedures;
   - progressive neurological disorder, other than MS, which may affect participation in the study or require the use of medications not allowed by the protocol
18. Patients unable to undergo MRI scans
19. Use of other investigational drugs at the time of enrolment or within the prior 30 days, or five elimination half-lives, or until the expected pharmacodynamic effect has returned to baseline, whichever is longer.
20. History of hypersensitivity to any of the study drugs or to drugs of similar chemical classes
21. Homozygosity for CYP2C9^{∗}3 (will be tested at Screening), or refusal to test for CYP2C9^{∗}3 haplotype
22. Patients using (or having used within four (4) weeks or 5 half- lives, whichever is greater before initial dosing) concomitant medications that are strong or moderate inducers of CYP2C9 (see Appendix 4).
23. Any other disease orf condition which could interfere with the participation in the study according to the study protocol, or with the ability with the patients to cooperate and comply with the study procedures.

### 2.3. Expanded Cardiac Monitoring Patients

In some patients who are eligible according to the above exclusion criteria may nevertheless certain potential risk factors for AV conduction suppression be present.

The "expanded cardiac monitoring group" includes all patients who meet the criteria outlined in Table 9 and who are therefore considered to either be at risk for serious bradyarrhythmia or who may tolerate bradycardia poorly. For the whole trial population (i.e. N = approximately 1530, see section 3), these patients will have an expanded cardiac monitoring scheme during the titration period according to section 5.d) below.

**Table 9 Expanded cardiac monitoring cardiovascular status criteria**

| |
|---|
| 1. Heart rate < 55 bpm at screening |
| 2. Cardiac conduction disorders such as incomplete left bundle branch block or second degree AV block Mobitz type I (Mobitz I) (either history or observed at screening) |
| 3. Minor ECG findings at screening PR interval: >200 msec and ≤230 msec; QRS duration ≤230 msec; QTcF >430 msec ≤450 msec (males); QTcF >450 msec ≤470 msec (females) |
| 4. History of or current cardiac disease such as heart failure NYHA class I, history of myocardial infarction > 12 months prior to enrollment. |
| 5. Any other condition which, in the opinion of the investigator, has a potential for AV conduction suppression and/or other risk factors that may require expanded cardiac monitoring |

### 2.4. Normal Cardiovascular Status Patients

Patients who don't meet the criteria outlined in Table 9 above are considered as having a normal cardiovascular status. It is noted that within at least approximately the first half of the study population, patients having a normal cardiovascular status will be required to conduct the extensive assessments required for the "Expanded Cardiac Monitoring Patients" according to section 5.d) below.

### 3. Study design

This is a randomized, multicenter, double-blind, placebo-controlled parallel-group study in approximately 1530 patients with SPMS. Patients are randomized (2:1) to receive either BAF312 or placebo.

The study has five potential epochs:
The Screening epoch consists of two phases, Screening phase (day -45 to day -8) and Baseline phase (day -7 to day -1). Patient eligibility is assessed during the Screening, and confirmed during Baseline.

The Double-blind treatment epoch starts at randomization. Patients are randomly assigned to either siponimod or placebo. Randomized patients begin with a six-day dose titration. Patients are monitored during the dose titration period. During the dose titration, all patients are expected to complete a study visit on the first day of dosing and on day 7. Patients have a study visit on Day 28 and then continue on a visit schedule every 3 months. The treatment duration for individual patients is variable (likely ranging from approximately 23 to 42 months) based on when the study-stop criteria are met.

The study is stopped when the required number of patients with 3-month confirmed disability progression is observed. In practice, the end of the study is planned in advance based on the number of events that have occurred at a given time point and a projection of when the required 374 events will occur. If the study cannot be completed in the predicted 42 months, then the end of study could alternatively be based on an absolute cut-off date regardless of the number of observed disability events. The maximum study duration for an individual patient is 60 months.

In addition to the Screening epoch and the Double-blind treatment epoch, there are three potential 'follow-up' epochs (Treatment discontinuation follow-up epoch, Follow-up epoch and Open-Label treatment epoch). The Treatment discontinuation follow-up epoch is the epoch used for patients who prematurely discontinue study medication and accept to stay in the study. Those patients follow an abbreviated schedule of assessments until the end of study.

The Follow-up epoch is used for patients who prematurely discontinue study medication and do not want to stay in the study in the abbreviated visits schedule. Those patients must complete a follow-up visit, one month after the end of treatment visit, as part of their Follow-up epoch. Those patients who complete the study on-drug and do not enter the extension must also complete the follow-up visit, one month after the end of treatment visit.

The Open-label treatment epoch is used only for patients who, under specific conditions when prematurely discontinuing the study drug, are offered open-label siponimod as rescue medication (following a specific informed consent procedure) while continuing to follow the normal schedule of assessments. Should these patients discontinue open label siponimod and discontinue study before end-of-study, they must also complete a follow-up visit assessment one month after the end of treatment visit.

The variable treatment duration design of this study means that the duration of treatment will range from approximately 23 months for the last enrolled patients up to approximately 42 months for the first enrolled patients. Thus, the majority (∼90%) of patients in the study will contribute more than 24 months of treatment. This longer-than-usual treatment duration addresses a recognized gap in current MS studies, which usually do not provide sufficient longer-term treatment data (>24months), which maybe particularly important for disability outcomes.

### 4. Treatment

### a) Investigational treatment

All patients start the treatment with a 5-day dose titration pack (0.25 mg, 0.25 mg, 0.5 mg, 0.75 mg, 1.25 mg) continuing to a 2 mg siponimod/placebo dose.

The study medication is packaged in blister cards for up-titration to the initial 2 mg/day dose (or for re-titration up to the 2 mg dose in case the patient misses four or more consecutive doses). The titration blisters contain tablets for a treatment period of 5 days. After the titration period, the study medication is then packaged in bottles and is administered orally once daily.

Both siponimod tablets and placebo tablets are identical in appearance and packaged in identical blisters and bottles.

### b) Dose titration/re-titration

All patients start with a dose titration to the initial dose of 2 mg/day. Patients follow the same dose titration procedures when restarting study drug after missing four or more consecutive doses during the trial. The patient is asked to record the time of drug intake in a patient diary during the 5-day titration period, including dosing day 6.

| Target dose | Day 1 | Day 2 | Day 3 | Day 4 | Day 5 |
|---|---|---|---|---|---|
| 2mg | 0.25 mg | 0.25 mg | 0.5 mg | 0.75 mg | 1.25 mg |

### Treatment arms

Patients are assigned to one of the following two treatment arms, siponimod 2 mg or placebo, in a ratio of 2:1.

### Treatment assignment, randomization (protocol standard text)

On day 1 (randomization visit), all eligible patients are randomized via Interactive Response Technology (IRT) to one of the treatment arms. After confirmation that the patient fulfils all the inclusion/exclusion criteria the IRT assigns a randomization number to the patient, which is used to link the patient to a treatment arm and specifies a unique medication number for the first package of investigational treatment to be dispensed to the patient.

### 5. Visit schedule and assessments

The baseline assessments are either carried out on the same day as randomization or on a preceding day up to 7 days before randomization, i.e. on day -7 to day 1 before the first drug administration.

Visit windows are as follows: ±7 days for day 28 and ±14 days for all following visits.

### a) Efficacy assessments

- Detailed neurological examination, including determination of Expanded Disability Status Score (EDSS) every 3 months
- Timed 25-Foot walk (T25W) every 3 months
- Nine Hole Peg Test (9-HPT) every 3 months
- Paced Auditory Serial Addition Test (PASAT) every 6 months
- MS Relapse - all relapses should be assessed while ongoing, and as soon as possible after onset, for confirmation.
- Magnetic Resonance Imaging (MRI) measures, including brain Magnetization Transfer Ration (MTR) and high resolution Tl-weighted imaging at selected sites
- Symbol Digital Modalities Test (SDMT) every 6 months
- Brief Visuospatial Memory Test-Revised (BVMT-R) every 6 months
- Low Contract Visual Acuity (LCVA) every 6 months

### b) Safety assessments

- Physical Examination includes assessment of skin, head and neck, lymph nodes, heart, lungs, abdomen, back, neurological function and comments on general appearance.
- Vital Signs include weight, height, sitting pulse rate, sitting systolic and diastolic blood pressure and oral temperature
- Laboratory evaluations
   - Hematology
   - Chemistry
   - Urinalysis
   - Serology
   - CYP2C9 Testing
- Electrocardiogram (ECG)
- Pregnancy assessment
- Chest High Resolution Chest tomography (HRCT)
- Pulmonary function test (PFT)
- Ophthalmologic examination
- Dermatology exam

### c) Other assessments

- Three Health related quality of life:
   - Multiple Sclerosis Impact Scale (MSIS-29)
   - Multiple Sclerosis Walking Scale (MSWS -12)
   - EuroQol (EQ-5D)
- Pharmacokinetics
- Optional expanded pharmacogenetics
- Exploratory biomarkers
- Colombia Suicide Severity Rating

### d) Cardiac monitoring

See section 2.3. and Table 9 above for a definition of the expanded cardiac monitoring group.

Sitting heart rate and blood pressure should be measured in triplicate before the first dose of BAF312, then hourly for 6 hours thereafter (by the Independent First Dose Administrator). When obtaining the pre-dose heart rate before the first dose, the patient should be allowed to sit and rest for 5 minutes before taking the sitting heart rate. Triplicate repeat sitting heart rate and blood pressure will then be made at 1-2 minute intervals. The sitting heart rate and blood pressure measurements should be taken as a baseline reading for both heart rate and blood pressure (before the first dose of BAF312). For comparison to the post-dose heart rate values, the lowest pre-dose value of heart rate should be used. Patients should receive the first dose of BAF312 before 12:00 PM (noon) in the outpatient setting.
1. In house (clinical/office/hospital) cardiac monitoring, of at least 6 hours, on Day 1 (treatment initiation):
   - Heart rate and blood pressure will be monitored in triplicate every hour for 6 hours
   - 12-lead ECG will be done before dosing, then at 3 hours post-dose and 6 hours post dose
   - Patients will only be discharged if they meet the following discharge criteria:
      - Heart rate must be at least 50 bpm or maximally 10 bpm lower than the baseline value
      - Heart rate must not be the lowest value measured during the observation period
      - Patients must have no symptoms related to decreased heart rate
      - ECG at 6 hours should not show any new significant treatment-emergent ECG abnormalities, other than sinus bradycardia, not observed at the patient's pre-dose ECG.
2. If the discharge criteria are not met at the 6-hour time point, monitoring beyond 6 hours will be performed until the discharge criteria are met.
3. Patients experiencing a clinically relevant treatment-related symptomatic event (e.g. chest pain, dizziness, palpitations, syncope, nausea and vomiting, etc) associated with reduction of the heart rate together with ECG changes at any time during the 6 hour monitoring period should be discontinued from treatment.
4. Patients experiencing a symptomatic event, (e.g. chest pain, dizziness, palpitations, syncope, nausea and vomiting) not associated with reduction of the heart rate and without ECG changes, which occur at any time during the 6-hour monitoring period may be discharged, provided they meet the discharge criteria, but must return on Day 2 for the second dose and will be re-monitored as on Day 1.
5. Mobile heart rate monitoring with telemetry, or a device with similar (or better) registration capability, will be used for continuous registration of heart rate and cardiac events, minimally 6 h post-dose, for Expanded Cardiac Monitoring patients during their outpatient phase of the BAF312 titration period. If MCT is not possible, Patient will be required to wear a Holter on Screening, Day 1 and Day 4 for 24 hours and for 6 hours on Day 7. The patients who will wear a Holter will need make the study visits to have the Holter machine attached.
6. On Day 4:
   a. For patients who will use the MCT device, the medical center or clinic will need to review the available MCT data for any findings. If there are any concerns, the medical center will need to contact the patient for further evaluation.
   b. For patients who will use the Holter machine, Patient visits the center prior to taking their dose and the site will need to apply the Holter. The patient will need to wear the Holter machine for 24 hours. Patient can remove the Holter device on the next day. The patient should bring with them the Holter device worn on day 1.
   c. If a bradyarrhythmia event is noted on the MCT, or the patient reports that they have experienced relevant symptoms e.g. chest pain, dizziness, palpitations, syncope, nausea and vomiting, the patient needs to go to the medical center for further evaluation and cardiac monitoring.
7. On Day 7, patients will return to the medical center or clinic to check the data from the mobile cardiac telemetry or other registration device and for follow-up evaluation. Heart rate and blood pressure will be monitored in triplicate predose and every hour for 6 hours and 12-lead ECG assessment conducted pre-dose, 3 hours post-dose and 6 hour post dose. The same discharge criteria (as described above) will apply. Patients who will use the Holter machine, will also need to wear the Holter on day 7 for 6 hours post dose.
8. Patients will receive written instructions on when to return to the clinic and a 24-hour contact phone number to call in the event of any new or relevant symptoms (chest pain, dizziness, palpitations, syncope, nausea and vomiting). These latter patients are instructed not to drive when they return to the clinic.

The assessment timings are provided in Table 10 below.

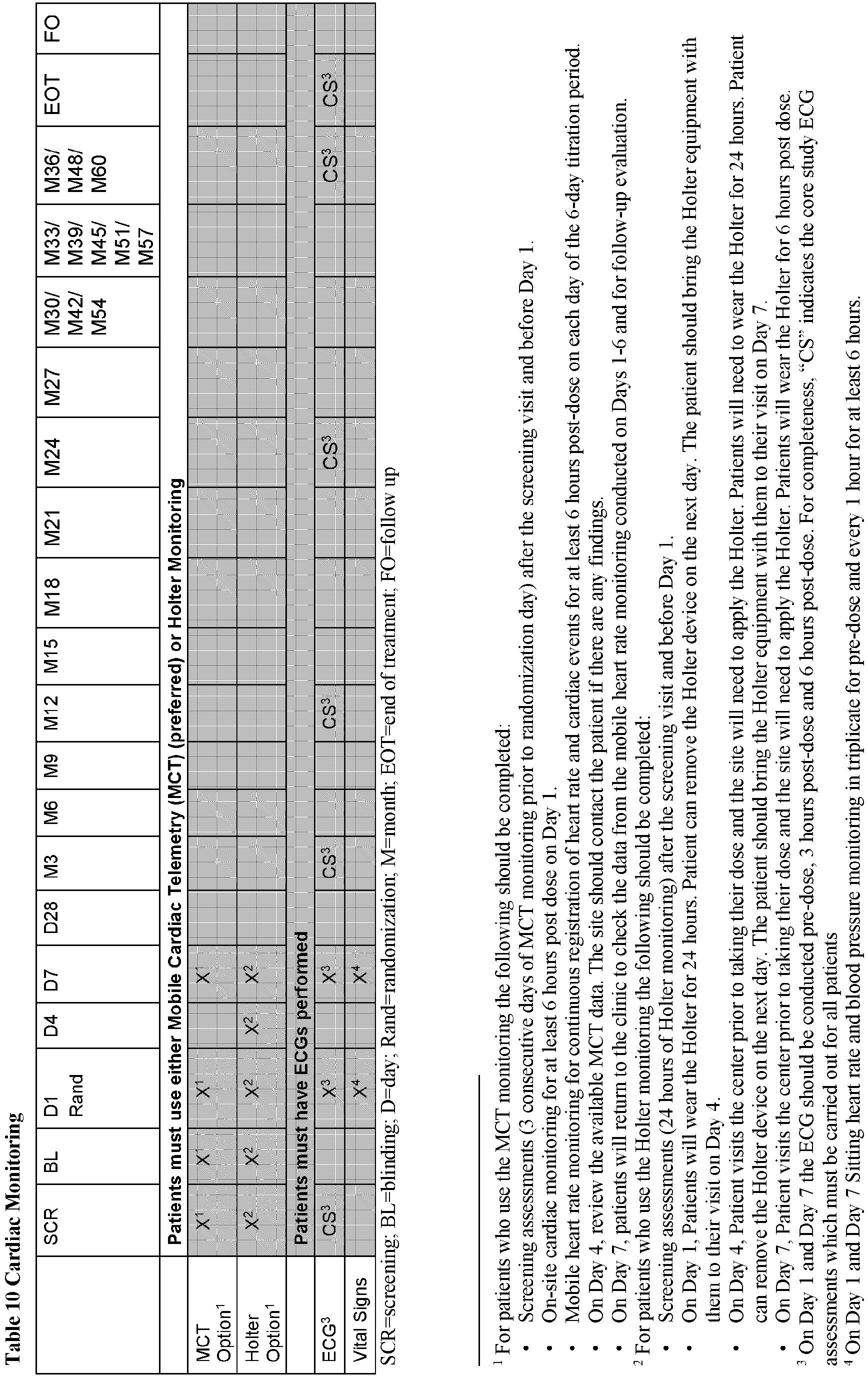

### 6. Results

Blinded cardiac monitoring data from approximately the first half of the patients (i.e. ½ of planned N=1530, see section 3 above) in the trial was assessed according to the expanded cardiac monitoring scheme outlined in section 5.d, Table 10. In other words, at the point in time when the assessment disclosed herein was made, **all** patients in the study (i.e. normal cardiovascular status patients and expanded cardiac monitoring patients) had undergone expanded first dose monitoring procedures, which include Holter or mobile cardiac telemetry (MCT) monitoring at screening/baseline and at dose initiation, pre-dose and hourly post-dose vital signs and 12- lead ECG at pre-dose, at 3 and 6 hours post-dose on Days 1 and 7. Data disclosed below is in blinded form, i.e. any patient mentioned below may have been on study drug or on placebo. Of the patients with data in the clinical database at the time point of the assessment, around 76.5% had normal cardiovascular status and around 23.5% fulfilled criteria (*vide supra*) for expanded cardiac monitoring.

Treatment initiation cardiac monitoring results were available as summarized below:
1. ECG post-dose - 691 patients (45.1% of planned to recruit)
2. MCT or Holter post-dose - 651 patients (42.5% of planned to recruit)
3. AE data for 775 patients (50.7% of planned to recruit)
4. SAE data for 862 patients (56.3% of planned to recruit)

The following surprising observations were made:
1. 12-lead ECG - no cases of 2nd degree AV block;
2. MCT - 2 patients with AV block 2nd degree Mobitz I on Days 3 and 4 (one in the expanded monitoring group, one - in normal);
3. Holter - 1 patient with AV block 2nd degree Mobitz I night-time episode <3 sec on Day 1 (expanded monitoring group);
4. 1 SAE of asymptomatic 2nd degree AV block Mobitz I in a patient with pre-existing AV block 1st degree and receiving carbamazepine (expanded monitoring group);
5. Cardiac disorders AEs reported in 54 (7%) of all patients; bradycardia in 17 (2.2%), sinus bradycardia in 8 (1.0%), AV block 2nd degree in 2 (0.3%) patients. No symptomatic bradyarrhythmia events.
6. No cases of symptomatic 2nd degree AV blocks.
7. No cases of 2:1 or Mobitz II AV blocks or high degree AV blocks.

Accordingly, the above data suggests that as a result of the particular dose titration regimen of the present invention using an immediate release dosage form of BAF312, post-dose cardiac monitoring in patients with normal cardiovascular status (definition: see section 2.4) may be discontinued. Strikingly, such a significant treatment simplification would benefit a large patient population. Based on the available data it is estimated that this population would be about 75% of the total patient population (see section 6, above).

To conclude, it has now been surprisingly found that an immediate release dosage form of siponimod can be used in the treatment of SPMS, with significantly reduced or even completely eliminated negative chronotropic side effects, when it is administered to patients who have experienced a specific titration regimen of siponimod. Accordingly, said titration regimen may allow for discontinuation of post-dose cardiac monitoring in subjects with normal cardiac status.

Embodiments of the invention are:
1. Siponimod for use in the treatment of an autoimmune disease,
   wherein an immediate release dosage form comprising 2 mg siponimod is administered once daily to a patient as a maintenance regimen, and
   wherein the patient has experienced a titration regimen beforehand of 0.25 mg siponimod at day 1, 0.25 mg at day 2, 0.5 mg at day 3, 0.75 mg at day 4 and 1.25 mg at day 5.
2. Siponimod for use according to embodiment 1, wherein the autoimmune disease is Secondary Progressive Multiple Sclerosis.
3. Siponimod for use in the treatment of Secondary Progressive Multiple Sclerosis according to embodiment 2, wherein the patient has experienced a titration regimen beforehand of one administration of 0.25 mg siponimod at day 1, one administration of 0.25 mg siponimod at day 2, one administration of 0.5 mg siponimod at day 3, one administration of 0.75 mg siponimod at day 4, and one administration of 1.25 mg siponimod at day 5.
4. Siponimod for use in the treatment of Secondary Progressive Multiple Sclerosis according to embodiment 3, wherein, in the titration regimen, immediate release dosage forms comprising 0.25 mg, 0.5 mg and 1 mg siponimod have been used.
5. Siponimod for use in the treatment of Secondary Progressive Multiple Sclerosis according to embodiment 2, wherein the immediate release dosage form shows an in-vitro release profile of siponimod of at least 80% after 30 minutes, measured according to USP app. II paddle, 900 ml, phosphate buffer and 0.1% (m/v) Tween 80, 60 rpm, 37°C.
6. Siponimod for use in the treatment of Secondary Progressive Multiple Sclerosis according to embodiment 4, wherein the immediate release dosage forms comprising 0.5 mg, 1 mg and 2 mg siponimod show an in-vitro release profile of siponimod of at least 80% after 30 minutes, measured according to USP app. II paddle, 500 ml, phosphate buffer and 0.1% (m/v) Tween 80, 60 rpm, 37°C; and
   wherein the immediate release dosage form comprising 0.25 mg siponimod show an in-vitro release profile of siponimod of at least 80% after 30 minutes, measured according to USP app. II paddle, 900 ml, phosphate buffer and 0.1% (m/v) Tween 80, 60 rpm, 37°C.
7. Siponimod for use in the treatment of Secondary Progressive Multiple Sclerosis according to embodiment 2, wherein the immediate release dosage form comprising 2 mg siponimod is such that the administration of a single dosage form leads in-vivo to a Cmax of 14.0 to 17.0 ng/ml and to a AUClast of 500 to 560 h·ng/ml.
8. Siponimod for use in the treatment of Secondary Progressive Multiple Sclerosis according to embodiment 4,
   wherein the immediate release dosage form comprising 2 mg siponimod is such that the administration of a single dosage form leads in-vivo to a Cmax of 14.0 to 17.0 ng/ml and to a AUClast of 500 to 560 h·ng/ml;
   wherein the immediate release dosage form comprising 1 mg siponimod is such that the administration of a single dosage form leads in-vivo to a Cmax of 5.5 to 9.5 ng/ml and to a AUClast of 200 to 320 h·ng/ml;
   wherein the immediate release dosage form comprising 0.5 mg siponimod is such that the administration of a single dosage form leads in-vivo to a Cmax of 3.0 to 4.8 ng/ml and to a AUClast of 100 to 160 h·ng/ml; and
   wherein the immediate release dosage form comprising 0.25 mg siponimod is such that the administration of a single dosage form leads in-vivo to a Cmax of 1.5 to 2.4 ng/ml and to a AUClast of 50 to 80 h·ng/ml.
9. Siponimod for use in the treatment of Secondary Progressive Multiple Sclerosis according to any one of embodiment s 2 to 8, wherein the dosage form of the maintenance regimen is a tablet comprising
   - 2 mg siponimod,
   - 0.5 to 10 mg moisture protective agent,
   - 0 to 25 mg disintegrant,
   - 15 to 200 mg filler.
10. Siponimod for use in the treatment of Secondary Progressive Multiple Sclerosis according to embodiment 9, wherein the moisture protective agent is selected from hydrogenated vegetable oil, castor oil, palmitol stearate, glyceryl palmitostearate and glyceryl behenate.
11. Siponimod for use in the treatment of Secondary Progressive Multiple Sclerosis according to any one of embodiment s 2 to 10, wherein the patients have EDSS scores of 3.0 to 6.5.
12. Siponimod for use in the treatment of Secondary Progressive Multiple Sclerosis by delaying disability progression,
   wherein an immediate release dosage form comprising 2 mg siponimod is administered once daily to a patient as a maintenance regimen, and
   wherein the patient has experienced a titration regimen beforehand of 0.25 mg siponimod at day 1, 0.25 mg at day 2, 0.5 mg at day 3, 0.75 mg at day 4 and 1.25 mg at day 5.
13. Siponimod for use in the treatment of Secondary Progressive Multiple Sclerosis by delaying disability progression according to embodiment 12, wherein the disability progression is measured as time to 3-month confirmed disability progression by EDSS compared to untreated patients, and wherein the time is increased by 10 to 75%.
14. Siponimod for use in the treatment of Secondary Progressive Multiple Sclerosis by delaying the worsening of impaired mobility,
   wherein an immediate release dosage form comprising 2 mg siponimod is administered once daily to a patient as a maintenance regimen, and
   wherein the patient has experienced a titration regimen beforehand of 0.25 mg siponimod at day 1, 0.25 mg at day 2, 0.5 mg at day 3, 0.75 mg at day 4 and 1.25 mg at day 5.
15. Siponimod for use in the treatment of Secondary Progressive Multiple Sclerosis by delaying the worsening of impaired mobility according to embodiment 14, wherein the worsening of impaired mobility is measured as time to 3-month confirmed worsening of impaired mobility of at least 20% from the baseline in the timed 25-foot walk test, and wherein the time is increased by 10 to 80%.
16. Siponimod for use in the treatment of Secondary Progressive Multiple Sclerosis by reducing the increase in T2 lesion volume,
   wherein an immediate release dosage form comprising 2 mg siponimod is administered once daily to a patient as a maintenance regimen, and
   wherein the patient has experienced a titration regimen beforehand of 0.25 mg siponimod at day 1, 0.25 mg at day 2, 0.5 mg at day 3, 0.75 mg at day 4 and 1.25 mg at day 5.
17. Siponimod for use in the treatment of Secondary Progressive Multiple Sclerosis by reducing the increase in T2 lesion volume according to embodiment 16, wherein the increase in T2 lesion volume is measured within 2 years of treatment, and wherein the increase is reduced by 10 to 100%.
18. A method of treating a patient with an autoimmune condition comprising administering an immediate release dosage form comprising 2 mg siponimod once daily to said patient as a maintenance regimen, and
   wherein said patient has experienced a titration regimen beforehand of 0.25 mg siponimod at day 1, 0.25 mg at day 2, 0.5 mg at day 3, 0.75 mg at day 4 and 1.25 mg at day 5.
19. A method of treating a patient with an autoimmune condition comprising administering an initial titration regimen of siponimod, and administering an immediate release dosage form comprising 2 mg siponimod once daily to said patient as a maintenance regimen, wherein said titration regimen comprises administering 0.25 mg siponimod at day 1, 0.25 mg at day 2, 0.5 mg at day 3, 0.75 mg at day 4 and 1.25 mg at day 5.
20. A method of ameliorating or preventing a negative chronotropic side effect associated with siponimod treatment of a patient with an autoimmune condition comprising administering an immediate release dosage form comprising 2 mg siponimod once daily to said patient as a maintenance regimen, and
   wherein said patient has experienced a titration regimen beforehand of 0.25 mg siponimod at day 1, 0.25 mg at day 2, 0.5 mg at day 3, 0.75 mg at day 4 and 1.25 mg at day 5.
21. A method of ameliorating or preventing a negative chronotropic side effect associated with siponimod treatment of a patient with an autoimmune condition comprising administering an initial titration regimen of siponimod, and administering an immediate release dosage form comprising 2 mg siponimod once daily to said patient as a maintenance regimen, wherein said titration regimen comprises administering 0.25 mg siponimod at day 1, 0.25 mg at day 2, 0.5 mg at day 3, 0.75 mg at day 4 and 1.25 mg at day 5.
22. A kit containing daily units of medication of siponimod of 0.25 mg, 0.25 mg, 0.5 mg, 0.75 mg and 1.25 mg, wherein the kit contains a dosage form comprising 0.25 mg siponimod.
23. A kit containing daily units of medication of siponimod of 0.25 mg, 0.25 mg, 0.5 mg, 0.75 mg and 1.25 mg, wherein the kit contains a dosage form comprising 0.25 mg siponimod for use as defined in embodiment 1.
24. Use of siponimod in the manufacture of a medication for the treatment of a patient with an autoimmune condition wherein an immediate release dosage form comprising 2 mg siponimod is administered once daily to said patient as a maintenance regimen, and wherein said patient has experienced a titration regimen beforehand of 0.25 mg siponimod at day 1, 0.25 mg at day 2, 0.5 mg at day 3, 0.75 mg at day 4 and 1.25 mg at day 5.
25. Siponimod for use in the treatment of an autoimmune disease,
   wherein an immediate release dosage form comprising 2 mg siponimod will be or is administered once daily to a patient as a maintenance regimen, and
   wherein the patient is experiencing or has experienced a titration regimen beforehand of 0.25 mg siponimod at day 1, 0.25 mg at day 2, 0.5 mg at day 3, 0.75 mg at day 4 and 1.25 mg at day 5.
26. Siponimod for use according to embodiment 25, wherein the autoimmune disease is Secondary Progressive Multiple Sclerosis.
27. Siponimod for use in ameliorating or preventing a negative chronotropic side effect associated with siponimod treatment of a patient with an autoimmune condition comprising administering an immediate release dosage form comprising 2 mg siponimod once daily to said patient as a maintenance regimen, and
   wherein said patient has experienced a titration regimen beforehand of 0.25 mg siponimod at day 1, 0.25 mg at day 2, 0.5 mg at day 3, 0.75 mg at day 4 and 1.25 mg at day 5.

## Claims

1. Siponimod for use in the treatment of an autoimmune disease,
wherein an immediate release dosage form comprising 2 mg siponimod is administered once daily to a patient as a maintenance regimen, and
wherein the patient has experienced a titration regimen beforehand of 0.25 mg siponimod at day 1, 0.25 mg at day 2, 0.5 mg at day 3, 0.75 mg at day 4 and 1.25 mg at day 5.

2. Siponimod for use according to claim 1, wherein the autoimmune disease is Secondary Progressive Multiple Sclerosis, especially wherein the patient has experienced a titration regimen beforehand of one administration of 0.25 mg siponimod at day 1, one administration of 0.25 mg siponimod at day 2, one administration of 0.5 mg siponimod at day 3, one administration of 0.75 mg siponimod at day 4, and one administration of 1.25 mg siponimod at day 5, more especially wherein, in the titration regimen, immediate release dosage forms comprising 0.25 mg, 0.5 mg and 1 mg siponimod have been used, more especially wherein the immediate release dosage forms comprising 0.5 mg, 1 mg and 2 mg siponimod show an in-vitro release profile of siponimod of at least 80% after 30 minutes, measured according to USP app. II paddle, 500 ml, phosphate buffer and 0.1% (m/v) Tween 80, 60 rpm, 37°C; and
wherein the immediate release dosage form comprising 0.25 mg siponimod show an in-vitro release profile of siponimod of at least 80% after 30 minutes, measured according to USP app. II paddle, 900 ml, phosphate buffer and 0.1% (m/v) Tween 80, 60 rpm, 37°C; yet more preferably wherein the immediate release dosage form comprising 2 mg siponimod is such that the administration of a single dosage form leads in-vivo to a Cmax of 14.0 to 17.0 ng/ml and to a AUClast of 500 to 560 h·ng/ml;
wherein the immediate release dosage form comprising 1 mg siponimod is such that the administration of a single dosage form leads in-vivo to a Cmax of 5.5 to 9.5 ng/ml and to a AUClast of 200 to 320 h·ng/ml;
wherein the immediate release dosage form comprising 0.5 mg siponimod is such that the administration of a single dosage form leads in-vivo to a Cmax of 3.0 to 4.8 ng/ml and to a AUClast of 100 to 160 h·ng/ml; and
wherein the immediate release dosage form comprising 0.25 mg siponimod is such that the administration of a single dosage form leads in-vivo to a Cmax of 1.5 to 2.4 ng/ml and to a AUClast of 50 to 80 h·ng/ml.

3. Siponimod for use in the treatment of Secondary Progressive Multiple Sclerosis according to claim 2, wherein the immediate release dosage form shows an in-vitro release profile of siponimod of at least 80% after 30 minutes, measured according to USP app. II paddle, 900 ml, phosphate buffer and 0.1% (m/v) Tween 80, 60 rpm, 37°C.

4. Siponimod for use in the treatment of Secondary Progressive Multiple Sclerosis according to claim 2, wherein the immediate release dosage form comprising 2 mg siponimod is such that the administration of a single dosage form leads in-vivo to a Cmax of 14.0 to 17.0 ng/ml and to a AUClast of 500 to 560 h·ng/ml.

5. Siponimod for use in the treatment of Secondary Progressive Multiple Sclerosis according to any one of claims 2 to 4, wherein the dosage form of the maintenance regimen is a tablet comprising
- 2 mg siponimod,
- 0.5 to 10 mg moisture protective agent,
- 0 to 25 mg disintegrant,
- 15 to 200 mg filler;
wherein the moisture protective agent is preferably selected from hydrogenated vegetable oil, castor oil, palmitol stearate, glyceryl palmitostearate and glyceryl behenate.

6. Siponimod for use in the treatment of Secondary Progressive Multiple Sclerosis according to any one of claims 2 to 5, wherein the patients have EDSS scores of 3.0 to 6.5.

7. Siponimod for use in the treatment of Secondary Progressive Multiple Sclerosis by delaying disability progression,
wherein an immediate release dosage form comprising 2 mg siponimod is administered once daily to a patient as a maintenance regimen, and
wherein the patient has experienced a titration regimen beforehand of 0.25 mg siponimod at day 1, 0.25 mg at day 2, 0.5 mg at day 3, 0.75 mg at day 4 and 1.25 mg at day 5, especially wherein the disability progression is measured as time to 3-month confirmed disability progression by EDSS compared to untreated patients, and wherein the time is increased by 10 to 75%.

8. Siponimod for use in the treatment of Secondary Progressive Multiple Sclerosis by delaying the worsening of impaired mobility,
wherein an immediate release dosage form comprising 2 mg siponimod is administered once daily to a patient as a maintenance regimen, and
wherein the patient has experienced a titration regimen beforehand of 0.25 mg siponimod at day 1, 0.25 mg at day 2, 0.5 mg at day 3, 0.75 mg at day 4 and 1.25 mg at day 5, especially wherein the worsening of impaired mobility is measured as time to 3-month confirmed worsening of impaired mobility of at least 20% from the baseline in the timed 25-foot walk test, and wherein the time is increased by 10 to 80%.

9. Siponimod for use in the treatment of Secondary Progressive Multiple Sclerosis by reducing the increase in T2 lesion volume,
wherein an immediate release dosage form comprising 2 mg siponimod is administered once daily to a patient as a maintenance regimen, and
wherein the patient has experienced a titration regimen beforehand of 0.25 mg siponimod at day 1, 0.25 mg at day 2, 0.5 mg at day 3, 0.75 mg at day 4 and 1.25 mg at day 5, especially wherein the increase in T2 lesion volume is measured within 2 years of treatment, and wherein the increase is reduced by 10 to 100%.

10. Siponimod for use in a method of ameliorating or preventing a negative chronotropic side effect associated with siponimod treatment of a patient with an autoimmune condition comprising administering an immediate release dosage form comprising 2 mg siponimod once daily to said patient as a maintenance regimen, and
wherein said patient has experienced a titration regimen beforehand of 0.25 mg siponimod at day 1, 0.25 mg at day 2, 0.5 mg at day 3, 0.75 mg at day 4 and 1.25 mg at day 5.

11. Siponimod for use in a method of ameliorating or preventing a negative chronotropic side effect associated with siponimod treatment of a patient with an autoimmune condition comprising administering an initial titration regimen of siponimod, and administering an immediate release dosage form comprising 2 mg siponimod once daily to said patient as a maintenance regimen, wherein said titration regimen comprises administering 0.25 mg siponimod at day 1, 0.25 mg at day 2, 0.5 mg at day 3, 0.75 mg at day 4 and 1.25 mg at day 5.

12. A kit containing daily units of medication of siponimod of 0.25 mg, 0.25 mg, 0.5 mg, 0.75 mg and 1.25 mg, wherein the kit contains a dosage form comprising 0.25 mg siponimod.

13. A kit containing daily units of medication of siponimod of 0.25 mg, 0.25 mg, 0.5 mg, 0.75 mg and 1.25 mg, wherein the kit contains a dosage form comprising 0.25 mg siponimod for use as defined in claim 1.

14. Use of siponimod in the manufacture of a medication for the treatment of a patient with an autoimmune condition wherein an immediate release dosage form comprising 2 mg siponimod is administered once daily to said patient as a maintenance regimen, and wherein said patient has experienced a titration regimen beforehand of 0.25 mg siponimod at day 1, 0.25 mg at day 2, 0.5 mg at day 3, 0.75 mg at day 4 and 1.25 mg at day 5.

15. Siponimod for use in the treatment of an autoimmune disease,
wherein an immediate release dosage form comprising 2 mg siponimod will be or is administered once daily to a patient as a maintenance regimen, and
wherein the patient is experiencing or has experienced a titration regimen beforehand of 0.25 mg siponimod at day 1, 0.25 mg at day 2, 0.5 mg at day 3, 0.75 mg at day 4 and 1.25 mg at day 5, especially wherein the autoimmune disease is Secondary Progressive Multiple Sclerosis.
